# EUROPEAN PATENT APPLICATION

(11) **EP 4 194 006 A1**
(43) Date of publication of application: **14.06.2023**
(21) Application number: 21854081.3
(22) Date of filing: 04.08.2021
(51) Int. Cl.: A61K 39/00, A61P 31/00, A61P 35/00, A61P 3/00

(54) **MRNA VACCINE COMPRISING ADJUVANT CAPABLE OF KINETIC CONTROL**

(30) Priority: 04.08.2020 KR 20200097552; 03.08.2021 KR 20210102220
(71) Applicant: PROGENEER INC., Guro-gu Seoul 08380 (KR)
(72) Inventor: LIM, Yong Taik, Seongnam-si, Gyeonggi-do 13597 (KR); PARK, Sei Hyun, Suwon-si, Gyeonggi-do 16421 (KR); PARK, Hye Min, Cheongju-si, Chungcheongbuk-do 28150 (KR); LEE, Chang Hoon, Suwon-si, Gyeonggi-do 16421 (KR)
(74) Representative: Dehns
(86) International application number: PCT/KR2021/010251
(87) International publication number: WO 2022/031021

(57) **Abstract**

The present invention relates to an mRNA vaccine comprising an adjuvant of which an immune activating function is kinetically controlled and, more specifically, to an mRNA vaccine comprising an adjuvant characterized in that, after mRNA is transcribed into proteins, the activating function of the adjuvant is sequentially active. The present invention relates to a key technology for optimizing the time interval between mRNA antigen expression and immune activation in order to effectively control antigen expression and antigen immunogenicity, which conflict. In order to optimize an mRNA antigen expression amount and the active time of an adjuvant, the present invention provides a key technology, which kinetically controls the action of an immune activating substance to increase the expression amount and immunogenicity of an antigen at the same time, and thus remarkably increases the efficacy of an mRNA vaccine.

## Description

### [Technical Field]

The present invention relates to an mRNA vaccine including an adjuvant whose immune activating function is kinetically controlled, and more particularly, to an mRNA vaccine in which the activating function of an adjuvant acts sequentially after mRNA is transcribed into a protein.

### [Background Art]

An mRNA vaccine is a drug used in prevention and treatment of a cancer, an infectious disease or an autoimmune disease using mRNA as an antigen. Compared with DNA vaccines, mRNA vaccines have advantages of being stable and facilitating mass production, and are expected to be widely used as platforms for anticancer vaccines and infectious disease vaccine in pandemic situations in the future (Korean Patent Publication No. 10-2020-0118386). However, fundamentally, an immune activating property inducing stimulation of adjuvant such as a toll-like receptor agonist (TLR agonist) of naked mRNA, on the contrary, is known to inhibit the expression of a sufficient level of mRNA antigen to exhibit pharmaceutical activity because it interferes with an mRNA translation signaling system. To solve this problem, an attempt to manufacture a vaccine using modified mRNA from which immunogenicity has been eliminated or attenuated is actively progressing. However, this case has a limit to effective induction of humoral immunity and/or cellular immunity due to low immunogenicity of mRNA. Therefore, it is very important to develop a novel system of mRNA vaccines that can exhibit the best effect by effectively regulating between the expression mechanism of an antigen and the mechanism showing the immunogenicity of an antigen, which works against each other.

Meanwhile, an immune response is a series of responses of activated immune cells against exogenous and endogenous materials, that is, antigens, and when microorganisms such as bacteria or viruses, or foreign bio-substances are introduced into the body, they are recognized by immune cells, which are then activated to secrete a factor such as a cytokine, thereby causing an inflammatory response. Recently, active research on mechanisms in an innate immune response stage that non-specifically acts at the early stage of infection is actively progressing, and among these mechanisms, a toll-like receptor (TLR), which is a gene capable of recognizing a pathogen in the early stage of inflammation, is known to recognize a cell membrane component of a pathogen or a nucleic acid component to induce an immune response, and by using the TLR, studies on various toll-like receptor ligands for activating immune cells are actively progressing.

Toll-like receptor agonists are agonists of toll-like receptors in endosome, and are known to effectively induce not only humoral immunity but also cellular immunity. However, such multifunctional toll-like receptor agonists are difficult to be dispersed in an aqueous solution due to their molecular structure. In addition, since they are dissolved only in specific organic solvents such as DMSO and methanol, and not dissolved in generally used organic solvents, there are limitations in manufacturing immune-activating drugs in various formulations. Therefore, a cream-type formulation (e.g., Aldara cream) in which various surfactants were mixed are commercialized. In some studies, to overcome the above problems, such an agonist is prepared in the form of a salt and thus can be dissolved in an aqueous solution. However, the toll-like receptor agonist prepared in the form of a salt is absorbed into the blood vessel in the body to induce an immune response in the blood vessel (systemic immune response), thereby causing many side effects (e.g., cytokine storm, various non-specific hypersensitive immune responses, etc.), so it is not easy to use. In addition, due to such side effects, to be actually used in treatment, it has to be treated at a smaller concentration than the effective dose, so it also becomes a factor in decreasing efficacy. To overcome such problems, some pharmaceutical companies are trying to prevent directly absorption into blood vessels by introducing a lipophilic lipid, or by direct chemical bonding to a polymer chain having a large size. However, since the toll-like receptor agonist prepared by such a method has its active site still exposed to the outside, it induces non-specific immune response in the body and still has the possibility of inducing toxicity.

Accordingly, if an mRNA vaccine including a toll-like receptor agonist in which an activation time is kinetically controlled and mRNA is developed, the immunogenic action of an mRNA antigen is inhibited at the early stage after administration, and instead, the expression of the mRNA antigen is normally induced. Sequentially thereafter, since the time interval between action mechanisms may be optimized by inducing the immune activation of mRNA as a toll-like receptor agonist, whose activation time is kinetically controlled, is converted to an activation state, this is expected to bring a great ripple effect in the next-generation mRNA vaccine market.

### [Disclosure]

### [Technical Problem]

To solve the problems of the related art described above, the present invention is directed to providing a composition for an mRNA vaccine, which includes an adjuvant whose activation time is kinetically controlled and mRNA as active ingredients, and more particularly, an mRNA vaccine composition which is able to increase an expression level of an antigen and simultaneously increase the immunogenicity of the antigen through immune activation induced by binding an adjuvant to an immune activation site in the endosome/lysosome and the cytosol after mRNA moves to the cytosol to cause translation.

However, technical problems to be solved in the present invention are not limited to the above-described problems, and other problems which are not described herein will be fully understood by those of ordinary skill in the art from the following descriptions.

### [Technical Solution]

The present invention provides a composition for an mRNA vaccine, which includes an mRNA antigen and a kinetically controllable immune modulator as active ingredients, in which the immune modulator is a complex having a cleavable linker bound to the active site thereof. The mRNA antigen is not limited as long as it is any mRNA antigen known to be used in an mRNA vaccine.

In one embodiment of the present invention, the composition for an mRNA vaccine induces the activation function of the immune modulator by cleaving the cleavable linker binding to the active site thereof after mRNA is translated into a protein after administration.

In another embodiment of the present invention, the kinetic control is characterized in that when a cleavable linker binds to the active site of an immune modulator to maintain an inactive state, and then the cleavable linker blocking the active site is cleaved within preferably 2 to 12 hours, and more preferably 3 to 9 hours after mRNA translation has begun, the activity of the immune modulator is temporally delayed. In addition, the kinetic control may operate on a molecular scale and/or macro scale.

In still another embodiment of the present invention, the cleavable linker may comprise any one or more bonds selected from the group consisting of a disulfide, a carbamate, a hydrazine, an ester, a peptide, an azide, an amide, a hydrazone, a thioether, a phosphodiester, a thioketal, and a combination thereof. However, there is no limitation to the type of linker as long as it can be cleaved by endogenous factors (enzyme, redox potential, GSH, pH, etc.) and/or exogenous factors (redox, pH, temperature, photo/light, magnetic, ultrasound, electrical responsive, etc.) in body.

In yet another embodiment of the present invention, the cleavable linker further includes an alkyl derivative such as ethylene oxide or ethylene glycol at one or both ends thereof, thereby increasing the solubility and flexibility of a complex in an aqueous solution.

In yet another embodiment of the present invention, in the cleavable linker, the chemical bond at the binding site is cleaved by any one or more factors selected from the group consisting of an enzyme, a pH, a redox potential, a temperature, ultrasonic waves, a magnetic force, and a light source.

In yet another embodiment of the present invention, to an end of the cleavable linker, any one or more materials selected from the group consisting of cholesterol, a lipid, a protein, an amino acid, a peptide and an oligonucleotide is bonded, and the material may be any one of various materials having a hydrophilic or lipophilic group, serving to block the active site of a toll-like receptor agonist.

In yet another embodiment of the present invention, the immune modulator is preferably a toll-like receptor agonist, more preferably, any one or more selected from the group consisting of a toll-like receptor 1 agonist, a toll-like receptor 2 agonist, a toll-like receptor 3 agonist, a toll-like receptor 4 agonist, a toll-like receptor 5 agonist, a toll-like receptor 6 agonist, a toll-like receptor 7 or 8 agonist, and a toll-like receptor 9 agonist.

In yet another embodiment of the present invention, the mRNA antigen and the kinetically controllable immune modulator may be loaded into any one or more drug delivery systems selected from the group consisting of a nanoliposome, a nanoemulsion, a nanomicelle, a hydrogel, a scaffold, a solid nanoparticle and a polymeric nanoparticle. The loading may be simply being contained within regardless of binding, inserting into a nanoparticle structure, or binding to the nanoparticle structure, but it is not limited as long as it is a form that includes the mRNA antigen and the immune modulator of the present invention.

In yet another embodiment of the present invention, after the mRNA antigen loaded in the drug delivery system is first delivered to the cytosol, the kinetically-controllable immune modulator interacts with a receptor on the cell surface or in an endosome or lysosome.

In yet another embodiment of the present invention, the drug delivery system may further include any one or more immune modulators selected from the group consisting of a toll-like receptor agonist, saponin, an antiviral peptide, an inflammasome inducer, a NOD ligand, a cytosolic DNA sensor (CDS ligand), a stimulator of interferon genes (STING) ligand, an outer wall component of a pathogen, alum, a lipid, a combination thereof, and a derivative thereof, and the immune modulator is not limited as long as it is an immune modulator that is used as an adjuvant.

In yet another embodiment of the present invention, the composition for an mRNA vaccine is used for preventing or treating any one or more diseases selected from the group consisting of an infectious disease, cancer, metabolic syndrome, an autoimmune disease, and a rare disease.

In addition, the present invention provides a method of preventing or treating an infectious disease, cancer, metabolic syndrome, an autoimmune disease, or a rare disease, which includes administering a composition for an mRNA vaccine including an mRNA antigen and a kinetically controllable immune modulator as active ingredients to a subject.

In addition, the present invention provides a composition for an mRNA vaccine for use in preventing or treating an infectious disease, cancer, metabolic syndrome, an autoimmune disease, or a rare disease, the composition including an mRNA antigen and a kinetically controllable immune modulator as active ingredients.

In addition, the present invention provides a use of a composition for an mRNA vaccine including an mRNA antigen and a kinetically controllable immune modulator as active ingredients for the manufacture of medicament for preventing or treating of an infectious disease, cancer, metabolic syndrome, an autoimmune disease, or a rare disease.

### [Advantageous Effects]

A composition for an mRNA vaccine according to the present invention can markedly improve a therapeutic effect of various mRNA vaccines using technology that can increase an expression level of an mRNA antigen and concurrently increasing the immunogenicity of an mRNA antigen through immune activation induced by binding mRNA to an immune activation site in an endosome/lysosome and the cytosol after moving to the cytosol to process translation, so it is expected to be applied to various mRNA vaccines.

### [Description of Drawings]

FIG. 1 is a schematic diagram of a novel idea that can improve both the antigen expression and immunogenicity of an mRNA vaccine by moving an mRNA antigen to the cytosol to process translation in an immature antigen-presenting cell stage, and sequentially interacting an adjuvant exhibiting a kinetic function with an immune activation site in the endosome/lysosome or cytosol.
FIG. 2 is a diagram illustrating a differentiated strategy of the present invention for developing an mRNA vaccine having optimal properties by effectively regulating antigen expression and antigen immunogenicity, which are opposite to each other, indicating that it is important to optimize the time interval between the expression of the mRNA antigen and the immune activation time.
FIG. 3 is a schematic diagram of an toll-like receptor agonist based adjuvant for controlling a kinetic immune function in an mRNA vaccine, showing that, to temporarily inactivate the immune active site (critical moiety), i.e., a material having a hydrophilic or lipophilic group binds to the active site of a toll-like receptor agonist for blocking, and a kinetic behavior can be regulated by linking a separable linker to the binding site.

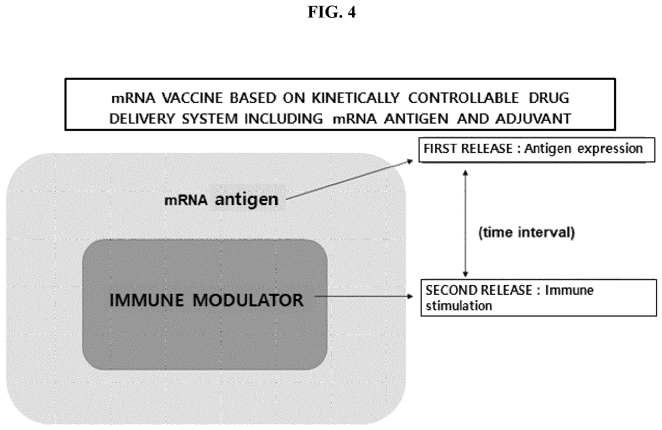

FIG. 4 is a diagram showing that the concept of kinetic control by a drug delivery system, designed to release an immune modulator to the endosome/lysosome and the cytosol after the mRNA antigen is first delivered to the cytosol, in which the drug delivery system may be prepared as nanoliposome, a nanoemulsion, a nanomicelle, a hydrogel, a scaffold, a solid nanoparticle and a polymeric nanoparticle.
FIG. 5 shows the result of confirming the degree of antigen presentation when BMDCs are treated with mRNA and R848 according to one embodiment of the present invention.
FIGS. 6 show the result of confirming the degree of cell activation by measuring a cell surface molecule when BMDCs are treated with mRNA and R848 according to one embodiment of the present invention.
FIGS. 7 show the result of confirming the secretion of an inflammatory cytokine when BMDCs are treated with mRNA and R848 according to one embodiment of the present invention.
FIG. 8 shows the result of confirming the degree of antigen presentation when BMDCs are treated with mRNA and LPS according to one embodiment of the present invention.
FIG. 9 shows the result of confirming the degree of antigen presentation when BMDCs are treated with mRNA and poly I:C according to one embodiment of the present invention.
FIG. 10 shows the result of confirming the secretion of a cytokine, type I interferon, when BMDCs are treated with mRNA and poly I:C according to one embodiment of the present invention.
FIGS. 11 show the result of confirming the degree of activation using the amount of IFN-γ+ T cells, and the levels of secreted IFN-γ and IL-2, when a co-culture of T cells and BMDCs is treated with mRNA and poly I:C according to one embodiment of the present invention.
FIGS. 12 show the result of confirming the degree of antigen presentation and the secretion of a cytokine, type I interferon, when BMDCs are treated with mRNA, poly I:C, and R848 according to one embodiment of the present invention.
FIGS. 13 show the result of confirming the degree of activation using the amount of IFN-γ+ T cells, and the levels of secreted IFN-γ and IL-2, when a co-culture of T cells and BMDCs is treated with mRNA poly I:C, and R848 according to one embodiment of the present invention.
FIGS. 14 show the result of confirming the degree of antigen presentation and the degree of cell activation when BMDCs are treated with mRNA, LPS, and R848 according to one embodiment of the present invention.
FIG. 15 shows the result of the degree of Th1 polarization when BMDCs are treated with mRNA, LPS, and R848 according to one embodiment of the present invention.
FIGS. 16 show the result of confirming the correlation between mRNA and type 1 IFN when BMDCs are treated with eGFP modified-mRNA, LPS, and R848 according to one embodiment of the present invention.
FIGS. 17 show the result of confirming the degree of antigen presentation when pDCs are treated with mRNA and R848, or mRNA and SKKU-078-liposome according to one embodiment of the present invention.
FIG. 18 shows the result of confirming the degree of cell activation when pDCs are treated with mRNA, or mRNA and SKKU-078-liposome according to one embodiment of the present invention.
FIGS. 19 show the result of confirming the phenotype of T cells when a co-culture of T cells and pDCs is treated with mRNA and SKKU-078-liposome according to one embodiment of the present invention.
FIGS. 20 show the result of confirming the dual phenotype of T cells and the secretion level of IFN-γ cytokine when a co-culture of T cells and pDCs is treated with mRNA and SKKU-078-liposome according to one embodiment of the present invention.

### [Best Modes of the Invention]

As a result of intensively studying a system capable of remarkably improving the effect of an mRNA vaccine by a co-administration method in which the expression time of an mRNA antigen and the immune activation time of an adjuvant are optimized, the present inventors invented a composition for an mRNA vaccine with significantly improved efficacy, in which a time interval between the expression time and immune activation time of the mRNA antigen is optimized using the mRNA antigen with a kinetically controllable immune modulator. The cleavable linker is connected to the active site of the adjuvant to make the activity of the adjuvant temporarily inactive after administered into the body. When the composition reaches targeted tissue or cells, the mRNA antigen begins to be translated into a protein, and then the adjuvant exhibits activity when a cleavable linker is cleaved within 2 to 12 hours.

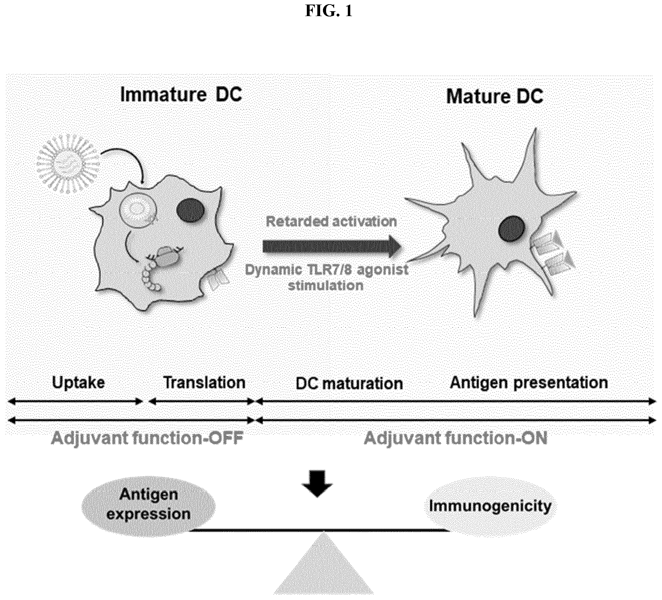

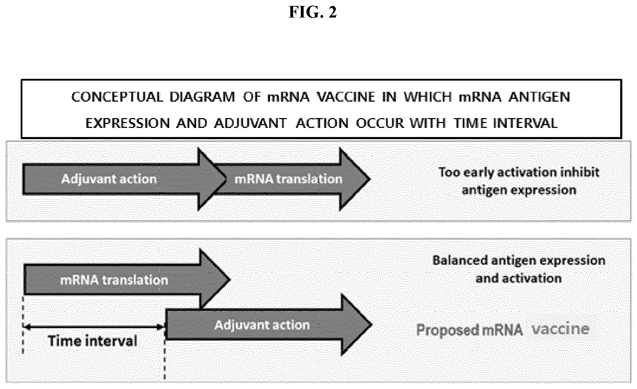

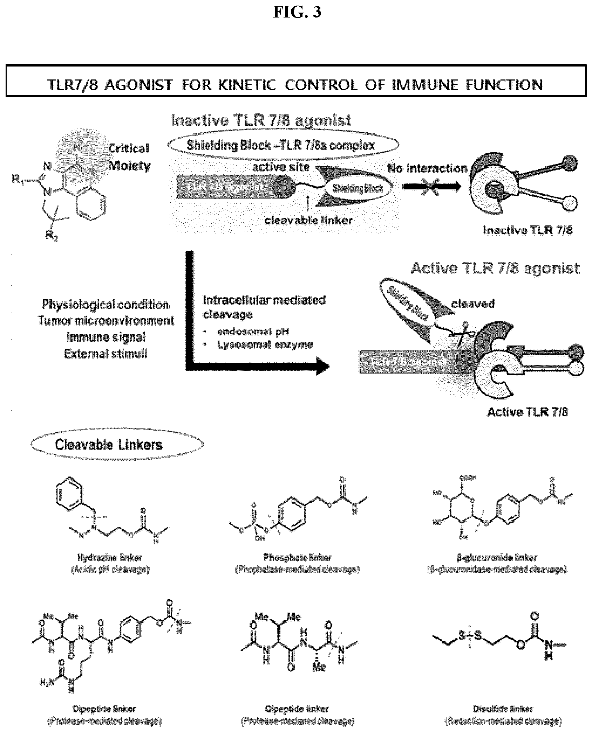

As shown in FIG. 1, the mRNA vaccine of the present invention is a system in which an mRNA antigen interacts with an immune activation site in the endosome/lysosome or cytosol after moving to the cytosol and being translated. Such a system is for solving the problem in which efficacy is weakened because the expression of a sufficient amount of the mRNA antigen serving as a vaccine is suppressed by disturbing the translation signaling system of the mRNA antigen due to activation of an immune activation action inducing the stimulation of a toll-like receptor after administered into the body, as a fundamental limitation of naked mRNA (FIG. 2). Conventionally, to solve the above-described problem, there were attempts to use modified mRNA with weakened immunogenicity, but as a result, due to low immunogenicity, a normal humoral immune response and/or cellular immune response is/are not induced, thereby weakening efficacy. To solve the above problem, the present inventors provided a new concept mRNA vaccine system, which optimizes the time interval between the expression time and immune activation time of an mRNA antigen in such a manner that when a cleavable linker is linked to the active site of a toll-like receptor agonist, the agonist remains temporarily inactive, and when the cleavable linker is cleaved in the endosome/lysosome or cytosol environment, the agonist is interact with a receptor to restore its activity (FIGS. 2 and 3).

The "mRNA vaccine" used herein is the generic term for vaccines using messenger ribonucleic acid (mRNA) containing genetic information as an antigen, and works on the principle that, when a vaccine is injected into the body, mRNA produces a protein, the human immune system senses it to induce an immune response, and then a neutralizing antibody is generated. Recently, such mRNA vaccines tend to be applied in not only infectious diseases but also various types of diseases, including cancer, autoimmune diseases, metabolic syndromes and rare diseases.

The "immune modulator" used herein is the generic term for materials serving to activate, induce, or restore a normal immune function of the immune system, and refers to a material that can be used as an adjuvant. The adjuvant is a material used together with an antigen for improving an immune response, that is, to increase antibody production, and increase humoral immunity and/or cellular immunity. Such immune modulators preferably include a toll-like receptor agonist, a saponin, an antiviral peptide, an inflammasome inducer, a NOD ligand, a cytosolic DNA sensor (CDS) ligand, a stimulator of interferon genes (STING) ligand, an emulsion, alum, incomplete Freund's adjuvant, Freund's adjuvant, and a combination thereof, and more preferably, a toll-like receptor agonist.

The "toll-like receptor agonist" used herein is a ligand directly or indirectly acting on a toll-like receptor, which is a membrane protein involved in innate immunity, and refers to a component that can cause a signaling response using a signaling pathway through the generation of an endogenous or exogenous ligand. The toll-like receptor agonist used herein may be a natural toll-like receptor agonist or a synthetic toll-like receptor agonist. The toll-like receptor agonist used herein may be a toll-like receptor 1 agonist, a toll-like receptor 2 agonist, a toll-like receptor 3 agonist, a toll-like receptor 4 agonist, a toll-like receptor 5 agonist, a toll-like receptor 6 agonist, a toll-like receptor 7 or 8 agonist, or a toll-like receptor 9 agonist.

The toll-like receptor 1 agonist refers to a ligand that can induce a signaling response by means of TLR-1, and may be, but is not limited to, a tri-acylated lipid peptide (LP); a phenol-soluble modulin; a lipid peptide of *Mycobacterium tuberculosis*; S-(2,3-bis(palmitoyloxy)-(2-RS)-propyl)-N-palmitoyl-(R)-Cys-(S)-Ser-(S)-Lys(4)-OH; a lipid peptide of *Borrelia burgdorfei*; or a trihydrochloride (Pam3Cys) lipid peptide mimicking an acetylated amino terminus of an OspA lipid peptide.

The toll-like receptor 2 agonist refers to a ligand that can induce a signaling response by means of TLR-2, and may be, but is not limited to, peptidoglycan, zymosan, HSP70, HMGB1, HA, or bam3Cys-Lip.

The toll-like receptor 3 agonist refers to a ligand that can induce a signaling response by means of TLR-3, and may be, but is not limited to, the poly(I:C) series, for example, poly(I:C), poly(ICLC), poly(IC12U), or Ampligen.

The toll-like receptor 4 agonist refers to a ligand that can induce a signaling response by means of TLR-4, and may be, but is not limited to, an outer membrane protein construct of *Shigella flexineri*, AGP, CRX-527, MPLA, PHAD, 3D-PHAD, GLA, or LPS.

The toll-like receptor 5 agonist refers to a ligand that can induce a signaling response by means of TLR-5, and may be, but is not limited to, flagellin.

The toll-like receptor 6 agonist refers to a ligand that can induce a signaling response by means of TLR-6, and may be, but is not limited to, a diacyl lipopeptide or lipoteichoic acid.

The toll-like receptor 7 or 8 agonist refers to a ligand that can induce a signaling response by means of TLR-7 or 8, and may be, but is not limited to, an imidazoquinoline-based agonist, a 8-hydroxyadenine-based agonist, a pteridone-based agonist, a 2-aminopyrimidine-based agonist, a benzoazepine-based agonist, or a 7-thia-8-oxoguanosine-based agonist, and the imidazoquinoline-based compound includes compound types disclosed in WO 2018 196823, WO 2011 049677, WO 2011 027022, WO 2017 102652, and WO 2019 040491, or pharmaceutically acceptable salts thereof, but the present invention is not limited thereto. In addition, the 8-hydroxyadenine-based compounds include compound types disclosed in WO 2012 080730, WO 2013 068438, WO 2019 036023, WO 2019 035969, WO 2019 035970, WO 2019 035971, WO 2019 035968, CN 108948016, US 2014 8846697, WO 2016 023511, WO 2017 133683, WO 2017 133686, WO 2017 133684, WO 2017 133687, WO 2017 076346, WO 2018 210298, WO 2018 095426, WO 2018 068593, WO 2018 078149, and WO 2018 041763, or pharmaceutically acceptable salts thereof, but the present invention is not limited thereto. The pteridone-based compounds include compound types disclosed in US 2010 0143301, WO 2016 007765, WO 2016 044182, WO 2017 035230, WO 2017 219931, WO 2011 057148, and CN 1087 94486, or pharmaceutically acceptable salts thereof, but the present invention is not limited thereto. The 2-aminopyrimidine-based compounds include compound types disclosed in WO 2010 133885, WO 2012066335, WO 2012 066336, WO 2012 067268, WO 2013 172479, WO 2012 136834, WO 2014 053516, WO 2014 053595, US 2018 0215720, WO 2012 156498, WO 2014 076221, WO 2016 141092, WO 2018 045144, WO 2015 014815, WO 2018 233648, WO 2014 207082, WO 2014 056593, WO 2018 002319 and WO 2013 117615, or pharmaceutically acceptable salts thereof, but the present invention is not limited thereto. The benzoazepine-based compounds include compound types disclosed in WO 2007 024612, WO 2010 014913, WO 2010 054215, WO 2011 022508, WO 2011 022509, WO 2012 097177, WO 2012 097173, WO 2016 096778, WO 2016 142250, WO 2017 202704, WO 2017 202703, WO 2017 216054, WO 2017 046112 and WO 2017 197624, or pharmaceutically acceptable salts thereof, but the present invention is not limited thereto. The 7-thia-8-oxoguanosine-based compounds include compound types disclosed in WO 2016 180691, WO 2016 055553, WO 2016 180743 and WO 2016 091698, or pharmaceutically acceptable salts thereof, but the present invention is not limited thereto. Other than the above, the toll-like receptor 7 or 8 compounds may also include toll-like receptor 7 or 8 compounds disclosed in PCT/US2009/035563, PCT/US2015/028264, PCT/US2016/020499, WO 2015 023598 and PCT/US 2015/039776, or pharmaceutically salt thereof. Alternatively, the toll-like receptor 7 or 8 agonists may include, but are not limited to, imiquimod, resiquimod, dactolisib, gardiquimod, sumanirole, motolimod, vesatolimod, loxoribine, SM360320, CL264, 3M-003, IMDQ, and Compound 54, and include all toll-like receptor 7 or 8 agonists which can be readily surmised by those of ordinary skill in the art.

The toll-like receptor 9 agonist refers to a ligand that can induce a signaling response by means of TLR-9, and may be, but is not limited to, an immune stimulating oligonucleotide. The immune stimulating oligonucleotide may include one or more CpG motifs, but the present invention is not limited thereto.

The "saponin" used herein is an amphipathic glycoside, and acts as a surfactant. For example, the saponin may be, but is not limited to, QS21, Quil A, QS7, QS17, β-escin, or digitonin.

The "antiviral peptide" used herein is the generic term for peptides exhibiting an antiviral effect, and may be, for example, KLK, but the present invention is not limited thereto.

The "inflammasome inducer" used herein is the generic term for materials that induce an inflammasome, which is a protein complex recognizing and activating a danger signal in the cytoplasm of a eukaryotic cell, and may be, for example, trehalose-6,6-dibehenate (TDB), but the present invention is not limited thereto.

The "NOD ligand" used herein is the generic term for ligands activating a Nod-like receptor, and may be, for example, M-TriLYS or N-glycosylated muramyl dipeptide, but the present invention is not limited thereto.

The "cytosolic DNA sensor (CDS) ligand" used herein is the generic term for ligands activating cGAS, which is a DNA sensor, and may be, for example, poly(dA:dT), but the present invention is not limited thereto.

The "stimulator of interferon genes (STING) ligand" used herein is the generic term for ligands activating STING, which is a sensor used by immune cells to detect cancer, and may be, for example, cGAMP, di-AMP, or di-GMP, but the present invention is not limited thereto.

The "cholesterol" used herein is a type of lipid, and is the generic term for steroid-based organic materials having a hydrophobic property, and the cholesterol may include various derivatives based on a cholesterol structure, and compounds that can be obtained by chemically changing a part of cholesterol. Preferably, the cholesterol includes bile acids (cholic acid, deoxycholic acid, lithocholic acid, and chenodeoxycholic acid), vitamin D, and steroid hormones (testosterone, estradiol, cortisol, aldosterone, prednisolone, and prednisone), but the present invention is not limited thereto. In addition, the cholesterol is a material that assists the toll-like receptor 7/8 agonist to be located on the surface and in various forms of nanoparticles, and may be replaced with lipid materials having a similar function thereto, for example, natural lipids such as a phospholipid, and synthetic lipids. Since the cholesterol serves to inactivate toll-like receptor 7 or 8 agonist when binding to its active site and prevents the toll-like receptor 7 or 8 agonist from being absorbed into a blood vessel in the body, there is no limitation as long as it is any type of well-known lipid.

The "cleavable linker" used herein includes a cleavable bond, and is the generic term for linkers in which cleavage can occur due to conditions such as low pH, enzymes or glutathione in the body such as a tumor microenvironment or the physiological environments of endosomes and lysosomes in cells; or external stimuli, that is, specific stimuli such as a temperature, redox potential, ultrasound, magnetic field, and near-infrared light. The cleavable linker preferably means a linker including a carbamate, disulfide, hydrazine, ester, peptide, or azide, but there is no limitation as long as it has a cleavable form.

The "prevention" used herein refers to all actions that inhibit diseases such as infectious diseases, cancer, metabolic syndrome, autoimmune diseases, and rare diseases or delay the onset thereof by administration of the composition according to the present invention.

The "treatment" used herein refers to all actions that are involved in improving or beneficially changing symptoms of infectious diseases, cancer, metabolic syndrome, autoimmune diseases, or rare diseases by administration of the composition according to the present invention.

The "individual or subject" used herein refers to a target to which the composition of the present invention can be administered, and there is no limitation to the target.

The "infectious disease" used herein is the generic term for diseases induced by infection caused by a heterogenous organism, such as a virus, a bacterium or a fungus.

The "cancer" used herein is the generic term for various blood cancers, malignant solid tumors, etc. which can expand locally by infiltration and systemically by metastasis. Although not particularly limited, specific examples of cancer include colorectal cancer, adrenal cancer, bone cancer, brain cancer, breast cancer, bronchial cancer, colon and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of cancer include adenocarcinoma, adenoma, basal cell carcinoma, cervical dysplasia and in situ carcinoma, Ewing's sarcoma, epidermoid carcinomas, giant cell tumors, glioblastoma multiforme, hairy-cell tumors, intestinal ganglioneuroma, hyperplastic corneal nerve tumors, islet cell carcinoma, Kaposi's sarcoma, leiomyoma, leukemias, lymphomas, malignant carcinoid, malignant melanomas, malignant hypercalcemia, marfanoid habitus tumors, medullary carcinoma, metastatic skin carcinoma, mucosal neuroma, myelodysplastic syndrome, myeloma, mycosis fungoides, neuroblastoma, osteosarcoma, osteogenic and other sarcoma, ovarian tumors, pheochromocytoma, polycythemia vera, primary brain tumors, small-cell lung tumors, squamous cell carcinoma of both ulcerating and papillary types, seminoma, soft tissue sarcoma, retinoblastoma, rhabdomyosarcoma, renal cell tumors or renal cell carcinoma, reticulum cell sarcoma, and Wilm's tumors. In addition, astrocytoma, gastrointestinal stromal tumors (GISTs), glioma or glioblastoma, hepatocellular carcinoma (HCC), and pancreatic neuroendocrine tumors are also included.

The "metabolic syndrome" used herein refers to a combination of three or more among five risk factors (high blood pressure, hyperglycemia, hypertriglyceridemia, low HDL cholesterol, and abdominal obesity) that increase health problems including heart disease, diabetes and stroke, occurring in one individual, and for example, includes metabolic diseases such as obesity, diabetes, high blood pressure, hyperlipidemia, heart disease, and gout. The metabolic syndrome is also the generic term for all diseases caused by metabolic syndrome.

The "autoimmune disease" used herein is the generic term for diseases caused by pathological responses to an autoantigen, and includes systemic autoimmune diseases such as systemic lupus erythematosus (SLE), rheumatoid arthritis (RA), and multiple sclerosis (MS), insulin-dependent diabetes mellitus (IDDM), Grave's disease, and allergies.

The "rare disease" used herein is the generic term for all diseases affecting only a small proportion of the population, which are usually hereditary, and generally refers to a disease that is difficult to diagnose due to its very low incidence or prevalence and has no proper treatment. Although each country has a slightly different definition, in Korea, in accordance with Article 2 of the Rare Disease Management Act, the rare disease is defined as "a disease in which the prevalence population is less than 20,000 or prevalence population is unknown because it is difficult to diagnose, determined according to the procedures and standards regulated by the ordinance of the Ministry of Health and Welfare." The World Health Organization (WHO) designates a rare disease as a case in which the prevalence is about 0.65 to 1 per 1,000 people, and the United States designates a rare disease as a case in which the total number of patients is less than 200,000, and Europe designates a rare disease as a case in which the total number of patients is 5 per 10,000 people.

The "pharmaceutical composition" or "vaccine composition" used herein may be prepared in the form of a capsule, tablet, granule, injection, ointment, powder, or drink, and the pharmaceutical composition or vaccine composition may be intended for humans. The pharmaceutical composition or vaccine composition may be, but is not limited to, formulated in the form of an oral formulation such as a powder, granules, a capsule, a tablet or an aqueous suspension, a preparation for external use, a suppository and a sterile injectable solution according to a conventional method. The pharmaceutical composition or vaccine composition of the present invention may include a pharmaceutically acceptable carrier. The pharmaceutically acceptable carrier may be a binder, a glidant, a disintegrant, an excipient, a solubilizer, a dispersant, a stabilizer, a suspending agent, a colorant, or a flavoring agent for oral administration, and for injection, a mixture of a buffer, a preservative, a pain relief agent, a solubilizer, an isotonic agent, and a stabilizer may be used, and for topical administration, a base, an excipient, a lubricant, and a preservative may be used. The pharmaceutical composition or vaccine compositions of the present invention may be prepared in various forms by being mixed with the above-described pharmaceutically acceptable carriers. For example, for oral administration, the pharmaceutical composition or vaccine composition of the present invention may be prepared in various dosage forms such as a tablet, a troche, a capsule, an elixir, a suspension, a syrup and a wafer, and for injectables, the pharmaceutical composition or vaccine composition of the present invention may be prepared in a unit dose ampoule or multiple dose forms. In addition, the pharmaceutical composition or vaccine composition of the present invention may be formulated as a solution, a suspension, a tablet, a capsule or a sustained-release preparation.

Meanwhile, the carrier, excipient, and diluent for preparation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. In addition, the carrier, excipient, and diluent for preparation may further include a filler, an anticoagulant, a lubricant, a wetting agent, a flavoring agent, an emulsifier, and a preservative.

The pharmaceutical composition or vaccine composition according to the present invention is administered, but not limited to, orally, intravenously, intramuscularly, intraarterially, intramedullary, intrathecally, intracardially, transdermally, subcutaneously, intraperitoneally, intranasally, enterally, topically, sublingually, or rectally. Oral or parenteral administration is preferable. The term "parenteral" used herein means subcutaneous, intravenous, intramuscular, intraarticular, intrabursal, intrasternal, intrathecal, intralesional and intracranial injections or infusions. The pharmaceutical composition or vaccine composition of the present invention may be administered in the form of a suppository for rectal administration.

The pharmaceutical composition or vaccine composition of the present invention may vary according to various factors including the activity of a specific compound used, age, body weight, general health, sex, diet, administration time, administration route, excretion rate, drug formulation, and the severity of a specific disease to be prevented or treated, and the dose of the pharmaceutical composition or vaccine composition may be selected by those of ordinary skill in the art according to a patient's condition and body weight, severity of a disease, a dosage form, an administration route and duration and may be administered daily at 0.0001 to 500 mg/kg or 0.001 to 500 mg/kg. The pharmaceutical composition or vaccine composition of the present application may be administered once a day or several times in divided portions. The dose does not limit the scope of the present application in any way. The pharmaceutical composition or vaccine composition according to the present invention may be formulated as a pill, a sugar-coated tablet, a capsule, a liquid, a gel, a syrup, s slurry or a suspension.

In addition, the vaccine composition according to the present invention may further include a conventionally known "adjuvant." Generally, the adjuvant is the generic term for materials that increase the humoral and/or cellular immune responses to an antigen, and may be any material that is known in the art without limitation. For example, immunity may be increased by further including Freund's complete adjuvant or incomplete adjuvant. In addition, if needed, the vaccine composition may be repeatedly administered for antigen stimulation, in addition to the initial dose.

Hereinafter, to help in understanding the present invention, exemplary examples will be suggested. However, the following examples are merely provided to more easily understand the present invention, and not to limit the present invention.

### [Examples]

### Example 1: Synthesis of a toll-like receptor 7/8 agonist-cholesterol complex

Various cholesterol-conjugated toll-like receptor 7 or 8 agonists (imidazoquinoline-based agonists, 8-hydroxyadenine-based agonists, pteridine-based agonists, 2-aminopyrimidine-based agonists, benzoazepine-based agonists, 7-thia-8-oxoguanosine-based agonists, etc.) were prepared by the chemical reaction of Scheme 1 or 2 below. More specifically, a temporarily-inactivated toll-like receptor 7 or 8 agonist-cholesterol complex was prepared by binding cholesterol (Sigma-Aldrich) to an amine (NH₂) site, which is the active site of the toll-like receptor 7 or 8 agonist, using a cleavable bond, such as a carbamate, disulfide, ester, peptide, or azide bond.

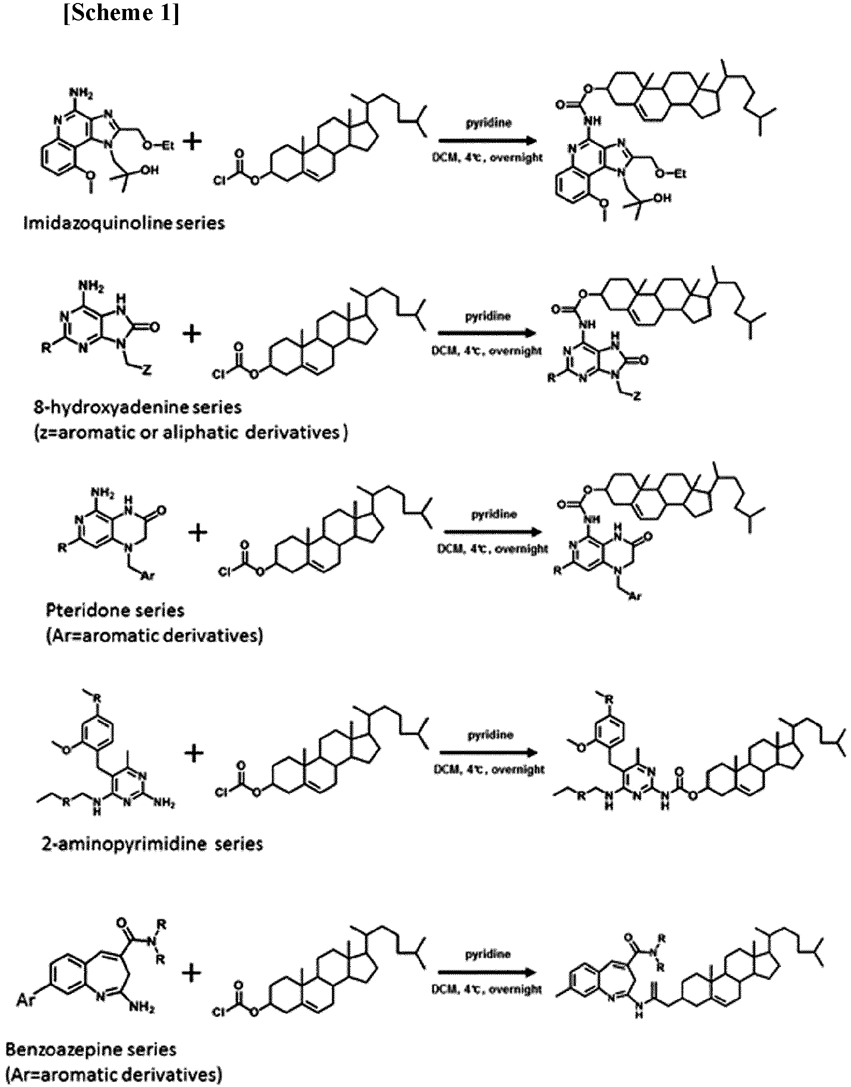

R is a side chain having an aliphatic or aromatic group, and may include-NH-, -CO-, -CONH-, -CSNH-, -COO-, -CSO-, -SO₂NH-, -SO₂-, -SO-, and -O-.

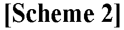

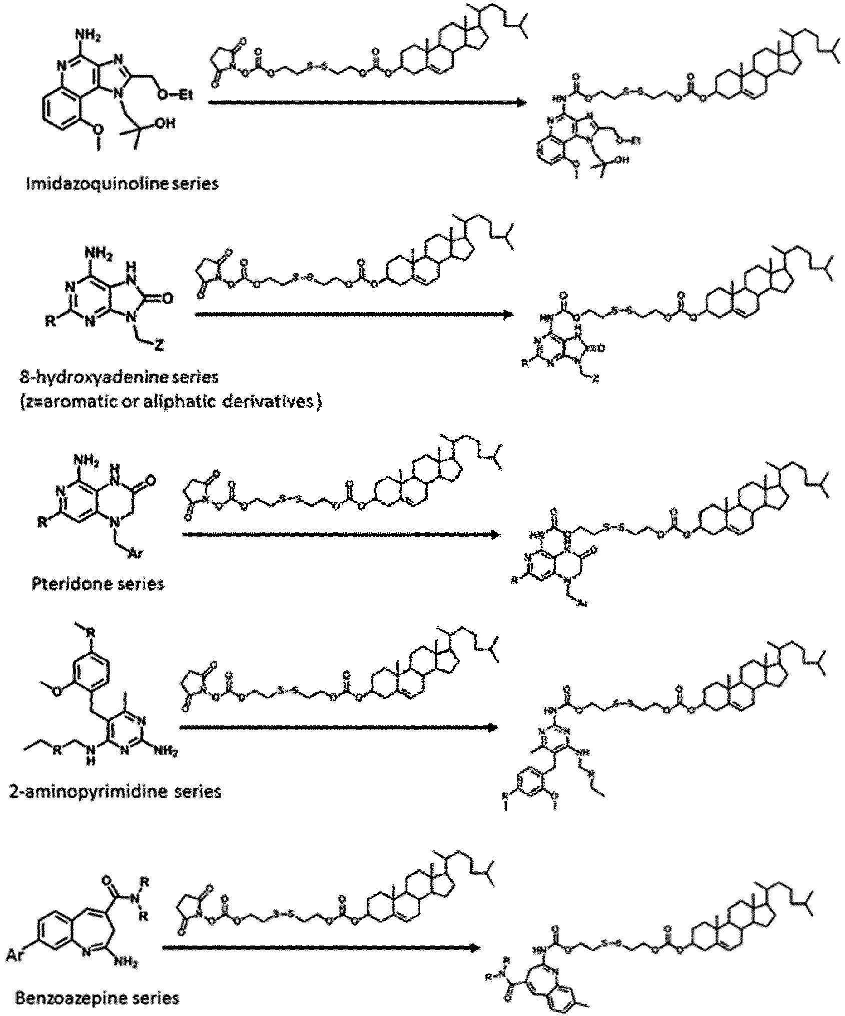

R is a side chain having an aliphatic or aromatic group, and may include-NH-, -CO-, -CONH-, -CSNH-, -COO-, -CSO-, -SO₂NH-, -SO₂-, -SO-, and -O-.

### Example 2: Synthesis of 2-methyl-1-(3-nitroquinolin-4- ylamino)propan-2-ol

2-Methyl-1-(3-nitroquinolin-4-ylamino)propan-2-ol (Compound 2) was synthesized using the method of Scheme 3 below. More specifically, 1-amino-2-methylpropan-2-ol (14 g) and tetraethylamine (9.6 g) were added to Compound 1 (30 g)-added dichloromethane (450 mL) at 10 to 20 °C and stirred for 2 hours, thereby preparing a mixture. Then, the mixture was concentrated by evaporating the solvent *in vacuo*, and then triturated with methyl tert-butyl ether (150 mL). The triturated mixture was separated using a filter and concentrated under low pressure, thereby obtaining Compound 2 (32 g, 85.2%, yellow solid). The structure of the obtained Compound 2 was verified using ¹H NMR.

¹H NMR (400 MHz, DMSO-d6): δ 9.91 (brs, 1H), 9.18 (s, 1H)), 8.46 (d, *J* = 8.0Hz, 1H), 7.83-7.92 (m, 2H), 7.56-7.60 (m, 1H), 5.15 (s, 1H), 3.86 (d, *J* = 4.8Hz, 2H), 1.15 (s, 6H).

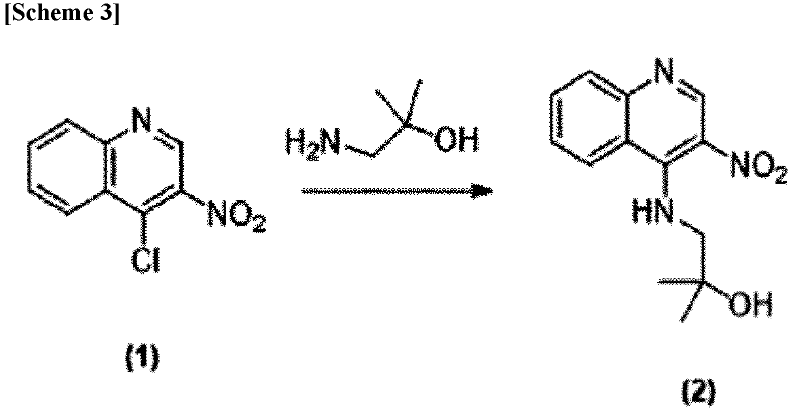

### Example 3: Synthesis of 1-(3-aminoquinolin-4-ylamino)-2-methylpropan-2-ol

1-(3-aminoquinolin-4-ylamino)-2-methylpropan-2-ol (Compound 3) was synthesized using the method of Scheme 4 below. In further detail, after mixing Compound 2 (32 g), methanol (500 mL) and Pd/C catalyst (3.2 g) in a reactor at 10 to 20 °C, the mixture was degassed and flushed three times using hydrogen. The hydrogen was vaporized to maintain 1 atm and the mixture was stirred at room temperature for 5 hours. Afterward, the mixture was triturated with methyl tert-butyl ether (100 mL). The triturated mixture was separated using a filter and concentrated at a low pressure, thereby obtaining Compound 3 (27 g, 95.4%, yellow solid). The structure of the obtained Compound 3 was verified using ¹H NMR.

¹H NMR (400 MHz, DMSO-d6): δ 8.37 (s, 1H)), 7.99-8.01 (m, 1H), 7.72-7.74 (m, 1H), 7.32-7.39 (m, 2H), 5.04 (s, 2H), 4.77 (brs, 1H), 4.67-4.70 (m, 1H), 4.12 (brs, 2H), 1.15 (s, 6H).

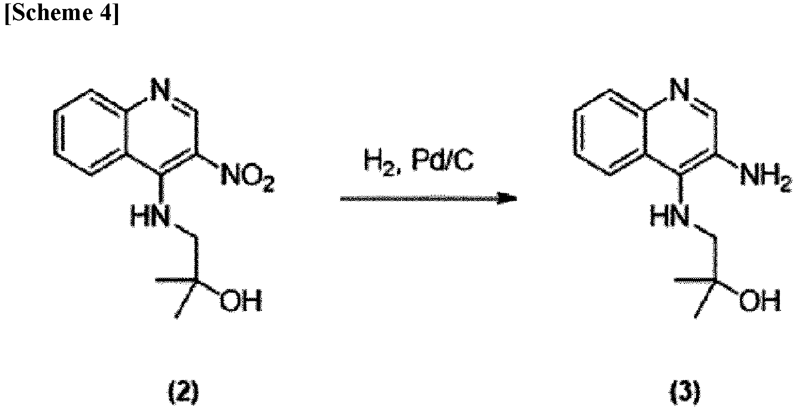

### Example 4: Synthesis of 1-(2-ethoxymethyl)-1H-imidazo[4,5-c]quinolin-1-yl)-2-methylpropan-2-ol

1-(2-ethoxymethyl)-1H-imidazo[4,5-c]quinolin-1-yl)-2-methylpropan-2-ol (Compound 4) was synthesized using the method of Scheme 5 below. In further detail, Compound 3 (27 g) and 2-ethoxyacetic acid (30 mL) were added to a reactor at 10 to 20 °C, and stirred at 120 to 130 °C for 5 hours. Afterward, the mixture was cooled to 20 to 25 °C, and then saturated sodium carbonate (150 mL) was added. Then, a reaction product was extracted using a mixed solution of dichloromethane and methanol (10/1, v/v). The extracted organic solution layer was washed with brine, and then moisture was removed over sodium sulfate (10 g). Then, the dehydrated organic solution layer was filtered using a filter and concentrated under low pressure, thereby obtaining Compound 4 (30 g, 85.8%, yellow gel). The structure of the obtained Compound 4 was verified using ¹H NMR.

¹HNMR (DMSO_d6 400 MHz): δ 9.18 (s, 1H)), 8.63 (d, *J* = 8.0Hz, 1H), 8.13 (dd, *J* = 1.6, 8.0Hz, 1H), 7.63-7.71 (m, 2H), 4.91 (s, 2H), 4.78 (brs, 2H), 3.54 (q, *J* = 6.8Hz, 2H), 1.10-1.18 (m, 9H).

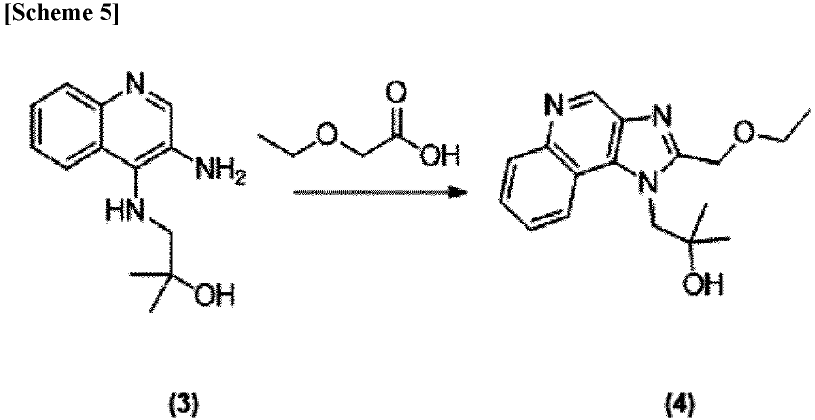

### Example 5: Synthesis of 2-(ethoxymethyl)-1-(2-hydroxy-2-methylpropyl)-1H-imidazo[4,5-c]quinolin 5-oxide

2-(ethoxymethyl)-1-(2-hydroxy-2-methylpropyl)-1H-imidazo[4,5-c]quinolin 5-oxide (Compound 5) was synthesized using the method of Scheme 6 below. In further detail, Compound 4 (30 g), dichloromethane (350 mL), and meta-chloroperoxybenzoic acid (26 g) were added to a reactor at 10 to 20 °C, and stirred at room temperature for 4 hours. Then, a saturated sodium carbonate solution (150 mL) and a sodium sulfate solution (150 mL) were added to the stirred mixture, and then a reaction product was extracted using a mixed solution of dichloromethane and methanol (10/1, v/v). The extracted organic solution layer was dehydrated over sodium sulfate (30 g), and filtered using a filter, followed by concentration under low pressure. Afterward, the concentrated reaction product was triturated using ethyl acetate (50 mL), separated using a filter, and then dried under low pressure, thereby obtaining Compound 5 (30 g, 94.9%, yellow solid). The structure of the obtained Compound 5 was verified using ¹H NMR.

¹HNMR (DMSO_d6 400 MHz): δ 9.04 (s, 1H)), 8.79 (d, *J* = 8.4Hz, 1H), 8.71 (d, *J* = 8.4Hz, 1H), 7.77-7.80 (m, 2H), 4.93 (s, 2H), 4.73 (brs, 2H), 3.54 (q, *J* = 6.8Hz, 2H), 1.12-1.18 (m, 9H).

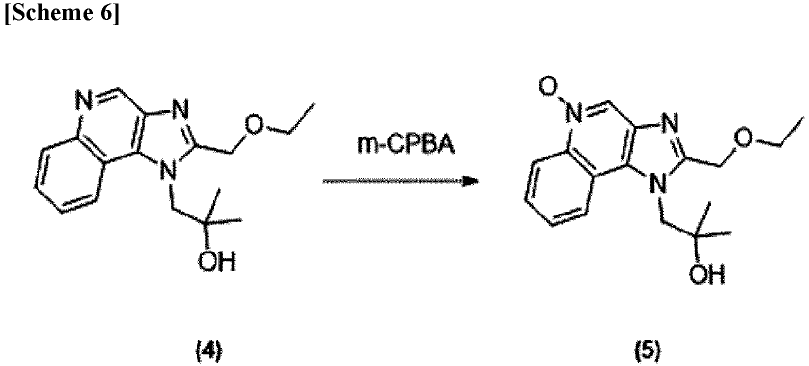

### Example 6: Synthesis of 1-(4-amino-2-ethoxymethyl)-1H-imidazo[4.5-c]quinolin-1-yl)-2-methylpropan-2-ol

1-(4-amino-2-ethoxymethyl)-1H-imidazo[4.5-c]quinolin-1-yl)-2-methylpropan-2-ol (Compound 6) was synthesized using the method of Scheme 7 below. In further detail, Compound 5 (30 g), DCM (600 mL), 4-methylbenzyl-1-sulfonyl chloride (18.2 g), and ammonia (NH₃.H₂O, 180 mL) were added to a reactor at 10 to 20 °C, and stirred at room temperature for 16 hours. Then, after adding distilled water to the stirred mixture, the mixture was separated using a mixed solution of dichloromethane and methanol (10/1, v/v). The separated organic solution layer was washed with brine and then dehydrated over anhydrous sodium sulfate (50 g). The dehydrated organic solution layer was filtered using a filter and then concentrated under low pressure, followed by triturating the concentrated reaction product using a mixed solution of methyl tert-butyl ether and methanol (15/1, v/v) for 30 minutes. Then, the resulting product was separated using a filter and dried under low pressure, thereby obtaining Compound 6 (18 g, 60%, yellow solid). The structure of the obtained Compound 6 was verified using ¹H NMR.

¹HNMR (DMSO_d6 400 MHz): δ 8.27 (d, *J* = 8.0Hz, 1H), 7.59 (d, *J* = 7.6Hz, 1H), 7.40 (t, *J* = 7.2Hz, 1H), 7.21 (t, *J* = 7.2Hz, 1H), 6.57 (brs, 2H), 4.89 (s, 2H), 4.68 (brs, 2H), 3.52 (q, *J* = 6.8Hz, 2H), 1.11-1.17 (m, 9H).

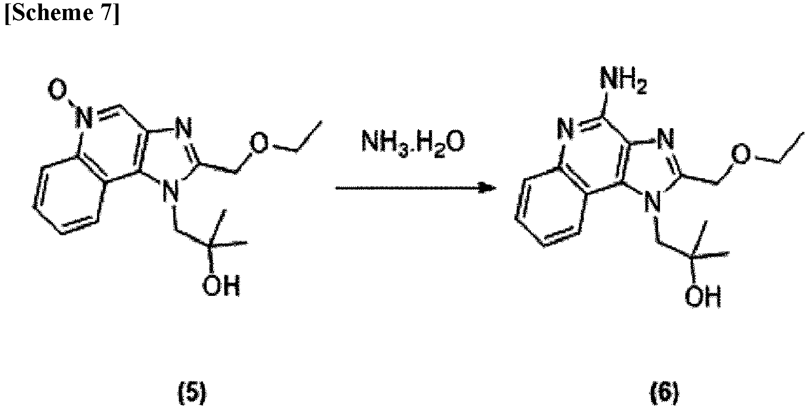

### Example 7: Synthesis of 10,13-dimethyl-17-(6-methylheptan-2-yl)-2,3,4,7,8,9,10,11,12,13,14,15,16,17-tetradecahydro-1H-cyclo[a]phenanthren-3-yl 2-(ethoxymethyl-1-(2-hydroxy-2-methylpropyl)-1H-imidazo[4,5-c]quinolin-4-yl carbamate

10,13-dimethyl-17-(6-methylheptan-2-yl)-2,3,4,7,8,9,10,11,12,13,14,15,16,17-tetradecahydro-1H-cyclo[a]phenanthren-3-yl 2-(ethoxymethyl)-1-(2-hydroxy-2-methylpropyl)-1H-imidazo[4,5-c]quinolin-4-yl carbamate (Compound 8) was synthesized using the method of Scheme 8 below. First, Compound 7 (TCI, 50 g, cholesterol chloroformate) was subjected to 250g-silica gel-filled column chromatography (0%~20% ethyl acetate in n-hexane), thereby obtaining pure Compound 7 (30 g). Then, Compound 6 (15 g) and dichloromethane (198.9 g) were added to a reactor at 10 to 20 °C, and the pure Compound 7 (30 g) and tetraethylamine (9.6 g) were sequentially added, followed by stirring at 20 to 25 °C for 16 hours. After adding water to the stirred mixture, dichloromethane was added to extract a reaction product. The extracted organic solution layer was washed with brine and dehydrated over anhydrous sodium sulfate (195 g). The dehydrated organic solution layer was filtered using a filter, and concentrated under low pressure. Then, the concentrated reaction product was triturated using a mixed solution of methyl tert-butyl ether and methanol (10/1, v/v). Afterward, Compound 8 (10.2 g, 55.1%, white solid) was obtained by separation using a filter and drying under low pressure. The structure of the obtained Compound 8 was verified using ¹H NMR. From the ¹H NMR result, it was confirmed that a complex in which resiquimod (R848) and cholesterol are connected via a carbamate bond was prepared.

¹H NMR (CDCl₃ 400 MHz): δ 8.13-8.19 (m, 2H), 7.59-7.63 (m, 1H)), 7.46-7.50 (m, 1H), 5.42-5.43 (m, 1H), 4.92 (brs, 2H), 4.72-4.80 (m, 3H), 3.68 (q, J = 6.8Hz, 2H), 3.24 (s, 1H), 2.51-2.59 (m, 1H), 2.36-2.47 (m, 1H), 1.96-2.11 (m, 3H), 1.81-1.95 (m, 2H), 1.45-1.75 (m, 9H), 1.02-1.35 (m, 27H), 0.94 (d, J = 6.4Hz, 3H), 0.89 (d, J = 6.4Hz, 6H), 0.71 (s, 3H).

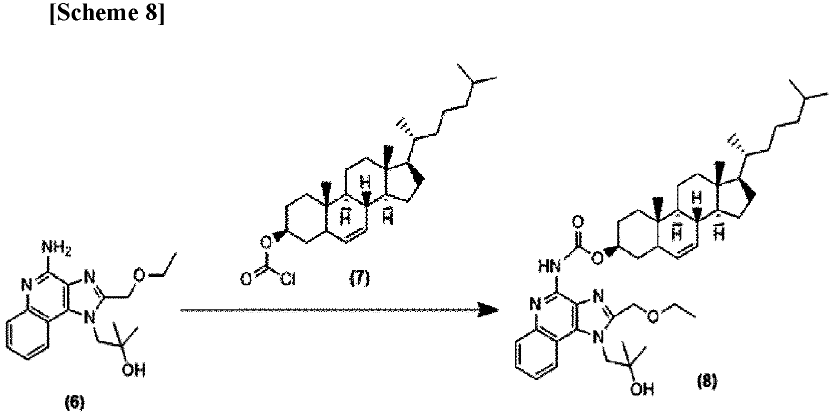

### Example 8: Synthesis of bis(2,5-dioxopyrrolidin-1-yl) 2,2'-disulfanediylbis(ethane-2,1-diyl) dicarbonate

Bis(2,5-dioxopyrrolidin-1-yl) 2,2'-disulfanediylbis(ethane-2,1-diyl) dicarbonate (Compound 9) was synthesized using the method of Scheme 9 below. First, Compound 7 (TCI, 70 g) was subjected to 350 g-silica gel-filled column chromatography (0%~20% ethyl acetate in n-hexane), thereby obtaining pure Compound 7 (40 g). Then, the pure Compound 7 (40 g) and dichloromethane (100 mL) were added to a reactor at 10 to 15 °C, and a bis(2-hydroxyethyl) disulfide-added dichloromethane (25 0mL) solution and pyridine (21 g) were sequentially added. The mixture was stirred at room temperature for 2 hours, and then distilled water (200 mL) was added. Subsequently, dichloromethane (150 mL) was added three times, thereby extracting a reaction product. The extracted organic solution layer was washed with brine and dehydrated over anhydrous sodium sulfate (20 g). The dehydrated reaction product was filtered using a filter, and then concentrated under low pressure. Afterward, Compound 9 (20 g, 39.6%, yellow gel) was obtained using column chromatography (silica gel, 300 g, 10%~30% ethyl acetate in n-hexane). The structure of the obtained Compound 9 was verified using ¹H NMR.

¹HNMR (CDCl₃ 400 MHz): δ 5.41-5.42 (m, 1H), 4.46-4.56 (m, 1H), 4.41 (t, *J* = 6.8Hz, 2H), 3.91 (t, *J* = 6.0Hz, 2H), 2.98 (t, *J* = 6.8Hz, 2H), 2.91 (t, *J* = 6.0Hz, 2H), 2.35-2.47 (m, 2H), 1.79-2.08 (m, 6H), 1.43-1.73 (m, 7H), 1.25-1.42 (m, 5H), 1.06-1.22 (m, 7H), 0.97-1.03 (m, 5H), 0.93 (d, *J* = 6.4Hz, 3H), 0.88 (dd, *J* = 1.6, 6.8Hz, 6H), 0.69 (s, 3H).

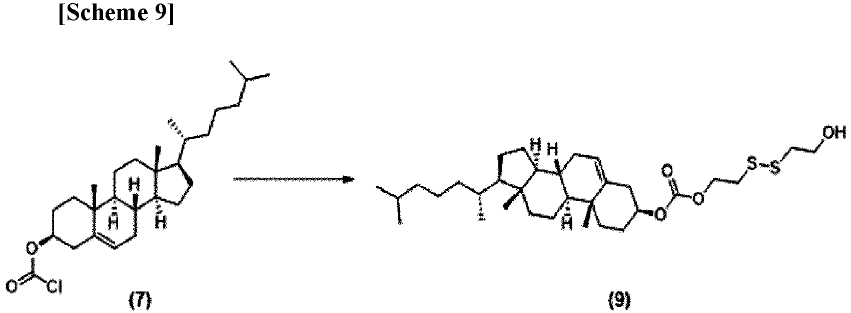

### Example 9: Synthesis of Compound 10

Compound 10 was synthesized using the method of Scheme 10 below. In further detail, Compound 9 (20 g) and dichloromethane (200 mL) were added to a reactor at 10 to 15 °C, and bis(2,5-dioxopyrrolidin-1-yl) carbonate (18 g) and tetraethylamine (10.7 g) were sequentially added. The mixture was stirred at room temperature for 3 hours, distilled water (300 mL) was added, and dichloromethane (150 mL) was then added three times to extract a reaction product. The extracted organic solution layer was washed with brine and dehydrated using anhydrous sodium sulfate (20 g). The dehydrated reaction product was filtered using a filter, and then the filtrate was concentrated under low pressure. Afterward, Compound 10 (18 g, 72%, yellow gel) was obtained using column chromatography (silica gel, 200 g, 5%~20% ethyl acetate in n-hexane). The structure of the obtained Compound 10 was verified using ¹H NMR.

¹HNMR (CDCl₃ 400 MHz): δ 5.39-5.40 (m, 1H), 4.57 (t, *J* = 6.8Hz, 2H), 4.43-4.52 (m, 1H), 4.37 (t, *J* = 6.4Hz, 2H), 2.96-3.03 (m, 4H), 2.84 (s, 4H), 2.34-2.44 (m, 2H), 1.78-2.04 (m, 5H), 1.42-1.73 (m, 7H), 1.22-1.40 (m, 5H), 1.06-1.20 (m, 7H), 0.95-1.04 (m, 5H), 0.91 (d, *J* = 6.0Hz, 3H), 0.86 (d, *J* = 6.4Hz, 6H), 0.67 (s, 3H).

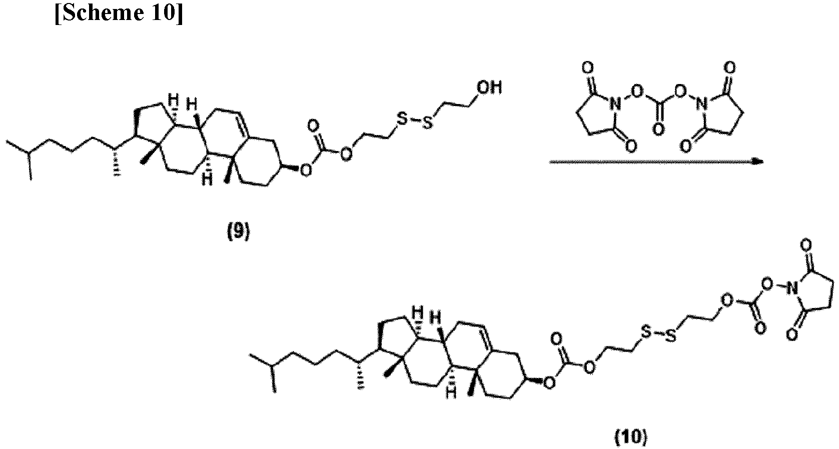

### Example 10: Synthesis of 2-((2-((10,13-diemthyl-17-(6-methylheptan-2-yl)-2,3,4,7,8,9,10,11,12,13,14,15,16,17-tetradecahydro-1H-cyclo[a]phenanthren-3-yloxy)cabonyloxy)ethyl)disulfanyl)ethyl 2-(ethoxymethyl)-1-(2-hydroxy-2-methylpropyl)-1H-imidazo[4,5-c]quinolin-4-yl carbamate

2-((2-((10,13-dimethyl-17-(6-methylheptan-2-yl)-2,3,4,7,8,9,10,11,12,13,14,15,16,17-tetradecahydro-1H-cyclo[a]phenanthren-3-yloxy)carbonyloxy)ethyl)disulfanyl)ethyl 2-(ethoxymethyl)-1-(2-hydroxy-2-methylpropyl)-1H-imidazo[4,5-c]quinolin-4-yl carbamate (Compound 11) was synthesized using the method of Scheme 11 below. In further detail, Compound 6 (15 g) and dichloromethane (198.9 g) were added to a reactor at 10 to 20 °C, and Compound 10 (40.5 g) and tetraethylamine (9.6 g) were sequentially added. The mixture was stirred at 20 to 25 °C for 16 hours, and then distilled water (225 mL) was added. Subsequently, dichloromethane (99.45 g) was added five times to extract a reaction product. The extracted organic solution layer was washed with brine and dehydrated over anhydrous sodium sulfate (195 g). Then, the dehydrated reaction product was filtered using a filter, and then the filtrate was concentrated under low pressure. Afterward, Compound 11 (10.8 g, 37.4%, white solid) was obtained using column chromatography (silica gel, 100 g, 10%~50% ethyl acetate in n-hexane). The structure of the obtained Compound 11 was verified using ¹H NMR. From the ¹H NMR result, it was confirmed that a complex in which R848 and cholesterol are cross-linked via disulfide was prepared.

¹H NMR (CDCl₃ 400 MHz): δ 8.15-8.17 (m, 2H), 7.60-7.64 (m, 1H)), 7.47-7.51 (m, 1H), 5.39-5.40 (m, 1H), 4.93 (s, 2H), 4.81 (s, 2H), 4.56 (t, *J* = 6.4Hz, 2H), 4.45-4.54 (m, 1H), 4.41 (t, *J* = 6.4Hz, 2H), 3.68 (q, *J* = 6.8Hz, 2H), 3.13 (s, 1H), 3.09 (t, *J* = 6.4Hz, 2H), 3.01 (t, *J* = 6.4Hz, 2H), 2.34-2.47 (m, 2H), 1.92-2.06 (m, 3H), 1.79-1.90 (m, 2H), 1.23-1.72 (m, 21H), 1.06-1.21 (m, 7H), 0.96-1.05 (m, 5H), 0.93 (d, *J* = 6.4Hz, 3H), 0.88 (dd, *J* = 1.6, 6.4Hz, 6H) , 0.69 (s, 3H).

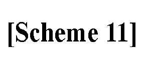

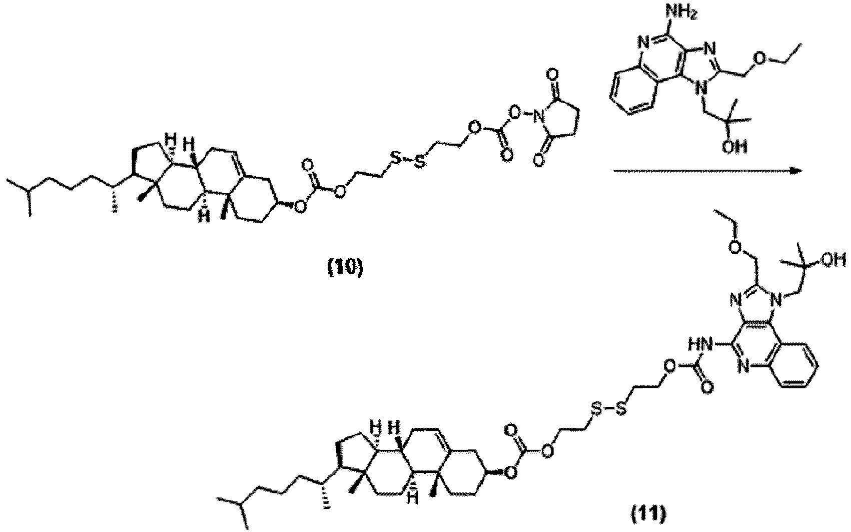

### Example 11: Preparation of nanoparticle including cholesterol-toll-like receptor 7 or 8 agonist complex

Since a cholesterol-toll-like receptor 7 or 8 agonist complex includes cholesterol, it may be easily prepared in various nanoparticle forms, and thus the interaction with immune cells may be maximized.

### 11.1. Preparation of nanoliposome including cholesterol-resquimod complex

To prepare an anionic nanoliposome including cholesterol-resquimod complex, 4 mg of 1,2-dioleoyl-sn-glycero-3-phosphocholine (DOPC, Avanti), 1.2 mg of cholesterol-conjugated resquimod, and 1 mg of 1,2-dipalmitoyl-sn-glycero-3-phospho-(1'-rac-glycerol) (DPPG, Avanti) were added and dissolved in 1 mL of chloroform, thereby preparing a mixture. The mixture was prepared in a thin film form by evaporating the solvent using a rotary evaporator, and 2 mL of phosphate-buffered saline was added to the thin film, and stirred at 45 °C for 30 minutes, thereby preparing an anionic nanoliposome including cholesterol-resquimod complex through homogenization using a tip sonicator (amplitude: 20%, 2 min). To prepare a cationic nanoliposome including cholesterol-resquimod complex, 4 mg of DOPC, 1.2 mg of cholesterol-resquimod complex, and 2 mg of dimethyloctadecylammonium bromide (DDAB) were added and dissolved in 1 mL of chloroform, thereby preparing a mixture. The mixture was prepared in a thin film form by evaporating the solvent using a rotary evaporator, and 2 mL of phosphate-buffered saline was added to the thin film, and stirred at 45 °C for 30 minutes, thereby preparing a cationic nanoliposome including cholesterol-resquimod complex through homogenization using a tip sonicator (amplitude: 20%, 2 min).

### 11.2. Preparation of nanoemulsion including cholesterol-resquimod complex

To prepare a nanoemulsion including cholesterol-resquimod complex, 1 mg of DOPC, 240 µg of cholesterol, and 240 µg of cholesterol-resquimod complex were added and dissolved in 1 mL of chloroform, thereby preparing a mixture. In addition, the mixture was prepared in the form of a thin lipid film by containing the mixture in a round-bottom flask and completely evaporating chloroform using a rotary evaporator. In addition, squalene (5 %v/v), Tween 80 (0.5 %v/v) and Span 85 (0.5 %v/v) were added and dissolved in 2 mL of phosphate-buffered saline, and the solution was added over the lipid film, dispersed using a tip sonicator for 1 minute, and stirred using a tube revolver for approximately 2 hours, thereby preparing a nanoemulsion including cholesterol-resquimod complex, and then stored at 4 °C in a refrigerator until use.

### 11.3. Preparation of nanomicelle consisting of cholesterol-resquimod complex and saponin

To prepare a nanomicelle consisting of cholesterol-resquimod complex and saponin, phosphatidylcholine, saponin and cholesterol-resquimod complex were mixed in a weight ratio of 5: 3: 2, and ether was added and dissolved in the mixture to have a concentration of 14 mg/mL, thereby preparing an ether solution including a lipid. In addition, saponin was dissolved in 4 mL distilled water at a concentration of 1.5 mg/mL, and contained in a 20 mL glass bottle. The glass bottle was closed with a rubber stopper, and then stored in a 55 °C water jacket. In addition, 1 mL of the lipid-containing ether solution was added to the glass bottle containing saponin at a rate of 0.2 mL/min using a syringe pump, and stirred for 2 hours. Here, the tip of a syringe needle was placed below the surface of the saponin-containing aqueous solution, and a second needle was inserted into the rubber stopper for ventilation. In addition, the glass bottle was transferred at room temperature, stirred for 3 hours to stabilize, thereby preparing a nanomicelle consisting of cholesterol-resquimod complex and saponin.

### 11.4. Preparation of polymer nanoparticle consisting of cholesterol-resquimod complex

60 mg of a PLGA polymer (Eudragit) having a composition ratio of lactide and glycolide of 50 : 50 was dissolved in 1 mL of a chloroform solvent. 5 mg of cholesterol-resquimod complex was added to the solvent and the cholesterol-resquimod complex and the polymer were dissolved using a sonicator (ultrasonic bath, Emerson Model CPX5800H-E). In addition, the resulting solution was added to 10 mL of a 2.5 % PVA aqueous solution by 200 µL, and dispersed for 1 minute using a tip sonicator (Sonics & Materials Model VCX 750). Here, the output of a disperser was 750 watts, a vibration intensity was 20 kHz, and an amplitude was set to 20%. In addition, to completely evaporate the PLGA-dissolved organic solvent, the prepared aqueous solution was stirred at 600 rpm and room temperature for 8 hours or more. To remove the unreacted polymer and cholesterol-resquimod complex, centrifugation was performed at 12,000 rpm for 12 minutes using a centrifuge (Hanil, Combi-514R), a supernatant was removed, 10 mL of deionized water was added, and then the resulting solution was dispersed in a sonicator for 30 seconds. The above-described process was repeated three times, followed by drying by lyophilization and storing at -20 °C.

### Example 12: Confirmation of efficacy of composition for mRNA vaccine including mRNA and cholesterol-toll-like receptor agonist complex

### 12.1. Method of treating composition for mRNA vaccine

To confirm the efficacy of the composition for an mRNA vaccine of the present invention, an experiment was performed using OVA mRNA which is widely used as an mRNA vaccine. In further detail, first, CleanCap^{®} OVA mRNA (5 moU, L-7210, TriLink Biotechnologies) and Opti-MEM^{™} (Reduced Serum Medium, ThermoFisher Scientific) were mixed at a volume ratio of 1:49, and Lipofectamine (Lipofectamine^{™} 2000 Transfection Reagent, ThermoFisher Scientific) and Opti-MEM^{™} were mixed at a volume ratio of 2:23 as a delivery system, and then the above mixtures were mixed again in a volume ratio of 1:1 and reacted for 5 minutes, thereby preparing an mRNA composition. As toll-like receptor agonists, a toll-like receptor 3 agonist, which is polyinosinic-polycytidylic acid (poly I:C, Sigma-Aldrich), a toll-like receptor 4 agonist, which is Lipopolysaccharide (LPS, Sigma-Aldrich), and a toll-like receptor 7 or 8 agonist, which is resiquimod (Biorbyt), were used. Poly I:C was used after being dispersed in phosphate buffered saline (PBS) at a concentration of 0.8 mg/mL, LPS was used after being dispersed in PBS at a concentration of 0.3 µg/mL, R848 was first dissolved in dimethyl sulfoxide (DMSO) at a concentration of 20 mg/mL, and then the dissolved solution was dispersed in PBS to a concentration of 20 µg/mL and used. In the case of treatment with a single toll-like receptor agonist, poly I:C was treated at 40 µg/mL, LPS was treated at 30 ng/mL, or R848 was treated at 1 µg/mL, and in the case of treatment with a combination of agonists, 2 µg/mL of poly I:C and 1 µg/mL of R848 were treated, or 2 ng/mL of LPS and 1 µg/mL of R848 were treated, and in both cases, mRNA was treated at 1 µg/mL.

### 12.2. Culture of bone marrow-derived dendritic cells, plasmacytoid dendritic cells, and splenocytes of ovalbumin-specific TCR-bearing mouse

To identify the efficacy of the composition for an mRNA vaccine of the present invention, as cells for experiments, bone marrow-derived dendritic cells (BMDCs), plasmacytoid dendritic cells (pDCs), and T cells isolated from the spleen of an ovalbumin-specific TCR-bearing mouse (OT-I transgenic mouse; OT-I mouse) were used. BMDCs and pDCs were obtained from the bone marrow of a laboratory mouse, C57BL/6 (female, Orient Bio). In further detail, a 6-weeks-old mouse was euthanized, the femur and tibia were cut, and then a medium was flushed to extract internal materials. In addition, the extracted materials were treated with a red blood cell lysis buffer (BioLegend) to remove red blood cells, and used. For pDCs, 6-weeks-old C57BL/6 was euthanized, the femur and tibia were cut, and then a medium was flushed to extract internal materials. The extracted materials were then treated with a red blood cell lysis buffer (BioLegend) to remove red blood cells, and only pDCs were isolated using a pDC isolation kit (Plasmacytoid Dendritic Cell Isolation Kit, mouse, MACS Miltenyi Biotec) to be used in the experiments. Spleen cells were used by extracting the spleen from an OT-I laboratory mouse, physically grinding the spleen to prepare single cells, washing the cells with PBS, and removing red blood cells with a red blood cell lysis buffer. The obtained cells were then seeded in a culture plate (Corning petri dish) with a size of 100 mm × 25 mm to 2 × 10⁶ cells, and cultured under conditions of 5% CO₂ and 37 °C. As a culture medium, a complete RPMI 1640 medium (Gibco) to which 20 ng/mL of a granulocyte-macrophage colony-stimulating factor (GM-CSF, Recombinant Mouse GM-CSF Protein, R&D Systems) was added, was used. During the culture period, the medium was replaced with a fresh medium every three days, and the cells on day 7 of culture were used for the experiments.

### 12.3. Co-culture of splenocytes of OT-I mouse and pDCs

To identify the efficacy of the composition for an mRNA vaccine of the present invention, T cells isolated from the spleen of an OT-1 mouse and pDCs were co-cultured. Splenocytes obtained in the same manner as in Example 12.2 (1×10⁵ cells/100 µL) and pDCs (1×10⁴ cells/100 µL) were seeded in 96-well plates, and treated with the composition for an mRNA vaccine prepared in the same manner as in Example 12.1, and then after 48 hours, cells and cytokines were analyzed. For analysis of intracellular cytokines, cells were treated with a cell activation cocktail (BioLgend) and a protein transport inhibitor (BD GolgiStop^{™} Protein Transport Inhibitor) before collecting the cells to activate the secretion of cytokines in the Golgi body in the cells, and then the cells were collected and measured. The collected cells were manipulated by perforating the cell membrane and cell using a Fixation/Permeabilization kit (BD Cytofix/Cytoperm^{™} Plus) to allow an antibody to be attached, and then the level of IFN-γ secreted in the cells was measured using IFN-γ antibodies (PE Rat Anti-Mouse IFN-γ; PE-IFN-γ, BioLegend).

### 12.4. Co-culture of splenocytes of OT-I mouse and BMDCs

To identify the efficacy of the composition for an mRNA vaccine of the present invention, T cells isolated from the spleen of an OT-1 mouse and BMDCs were co-cultured. To perform *ex vivo* DC therapy (cell therapy), before the co-culture with the splenocytes, first, the BMDCs were treated with a composition for an mRNA vaccine prepared in the same manner as in Example 12.1 and cultured for 24 hours. And then BMDCs (1 × 10⁴ cells/100 µL) cultured for 24 hours and T cells (1 × 10⁵ cells/100 µL) were seeded in 96-well plates, and the cells and cytokines were analyzed after 48 hours. The cytokine analysis was performed in the same manner as in Example 12.3, and to measure the degree of BMDC activation, the mean fluorescence intensities (MFIs) of a cluster of differentiation 80 (CD80), CD86 and CD40, which are cell surface molecules, were confirmed by attaching a fluorescent antibody. The CD80 measurement used PerCP/Cyanine5.5 anti-mouse CD80 antibody (PerCP/Cy5.5-CD80, BioLegend), and CD86 was confirmed using PE anti-mouse CD86 antibody (PE-CD86, BioLegend), and CD40 was confirmed using FITC anti-mouse CD40 antibody (FITC-CD40, BioLegend). For the BMDCs, measurement was performed by single cell isolation through regulation of scattering light (forward scatter and side scatter; FSC and SSC), and isolating a BMDC population using CD11c antibodies (APC anti-mouse CD11c antibody (APC-CD11c), BD Biosciences). To measure the degree of antigen presentation, anti-OVA SIINFEKL antibodies were used.

### 12.5. Confirmation of efficacy of composition for mRNA vaccine in BMDCs

### 12.5.1. Effect of single toll-like receptor agonist

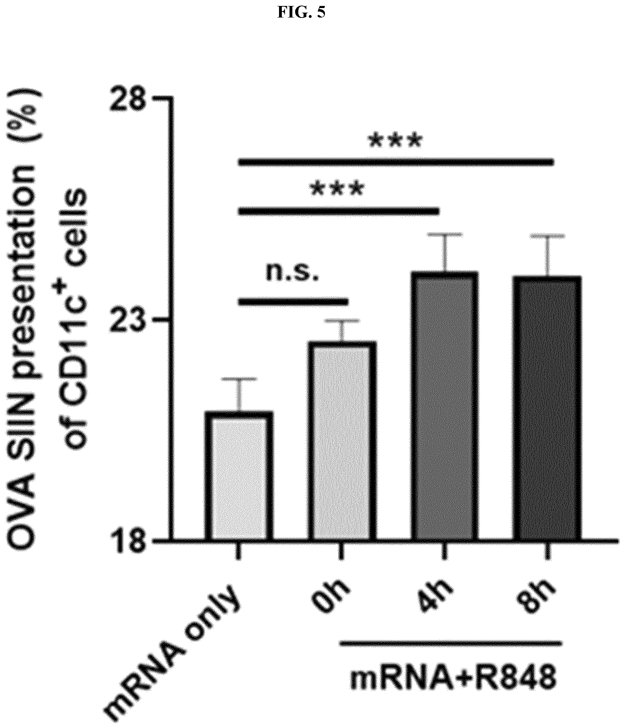

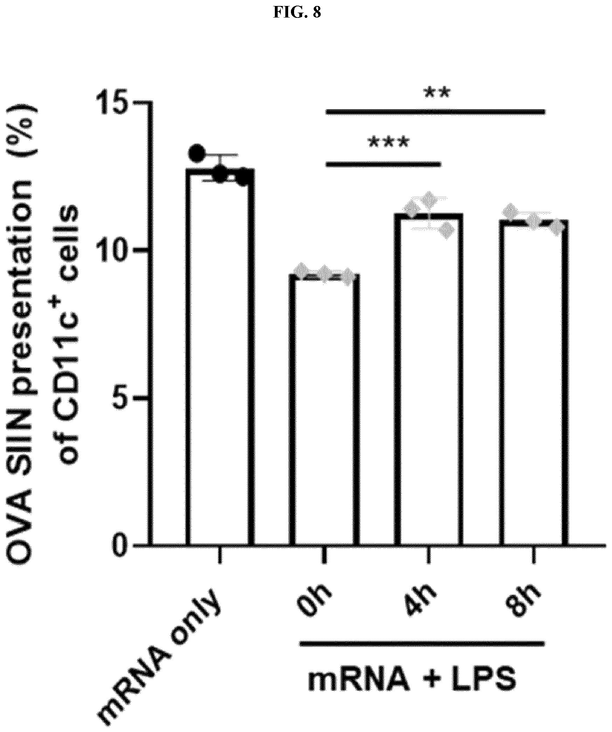

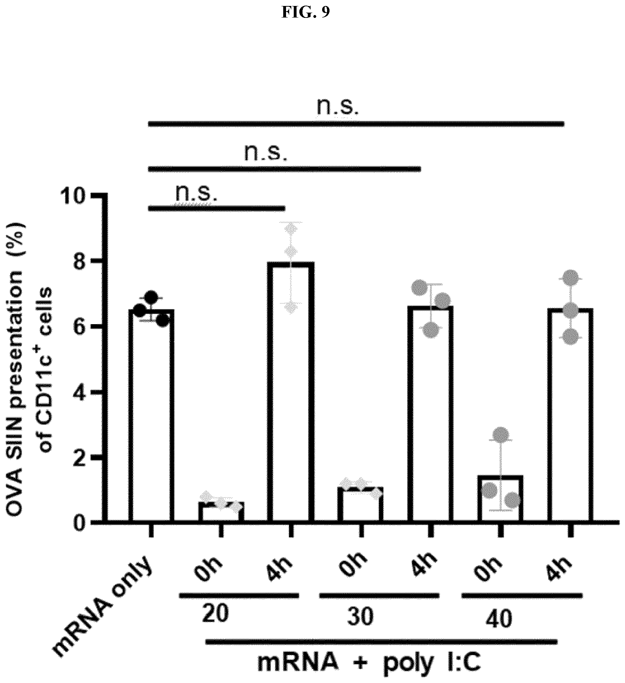

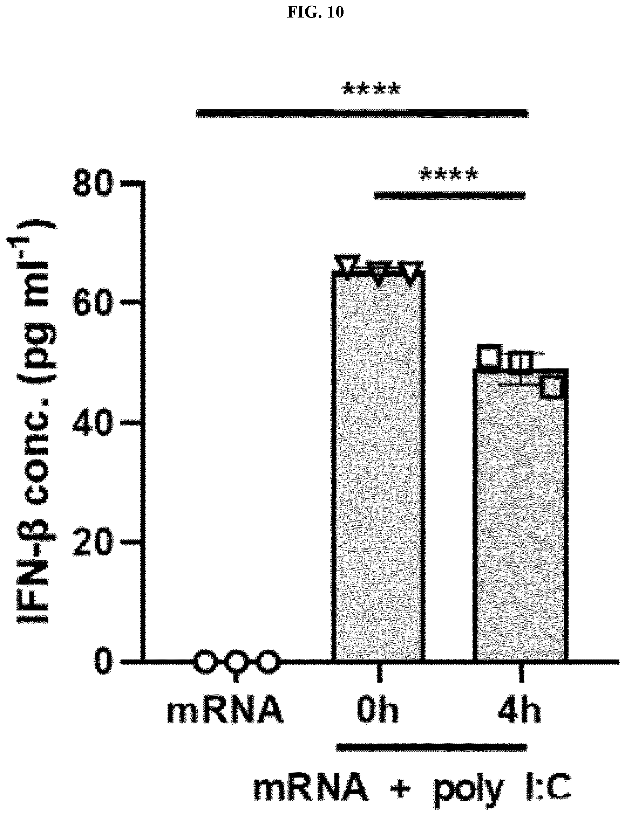

To confirm the effect of a composition for an mRNA vaccine including a single toll-like receptor in BMDCs, first, BMDCs were treated with a toll-like receptor agonist and modified-mRNA (5-methoxyuridine modified OVA mRNA, TriLink) in the same manner as in Example 12.1, and an antigen presentation degree, amounts of cell surface molecules and levels of cytokines such as interleukin-12p70 (IL-12p70) and interferon were measured. The levels of inflammatory cytokines were measured using ELISA. As toll-like receptor agonists, toll-like receptor 7 or 8 agonist R848, toll-like receptor 4 agonist LPS, and toll-like receptor 3 agonist poly I:C were used, poly I:C was used at different concentrations, for example, 20 µg/mL, 30 µg/mL, and 40 µg/mL. Afterward, all experiments were repeated at least in triplicate, and the result was expressed as mean ± standard deviation (SD). Statistical significance was confirmed by the Student's t-test, and when P < 0.05, it was determined that there is statistical significance. The result using R848 is shown in FIGS. 5 to 7, the result using LPS is shown in FIG. 8, and the result using poly I:C is shown in FIGS. 9 and 10.

As shown in FIG. 5, compared to a control treated with mRNA alone (mRNA only), it was confirmed that in an experimental group (0h) treated with mRNA and R848 at the same time, the degree of antigen presentation is partially increased, but there was no significant difference, and in an experimental group (4h) treated with mRNA and treated with R848 four hours later and an experimental group (8h) treated with mRNA and treated with R848 eight hours later, there was a significant difference and the degree of antigen presentation increased.

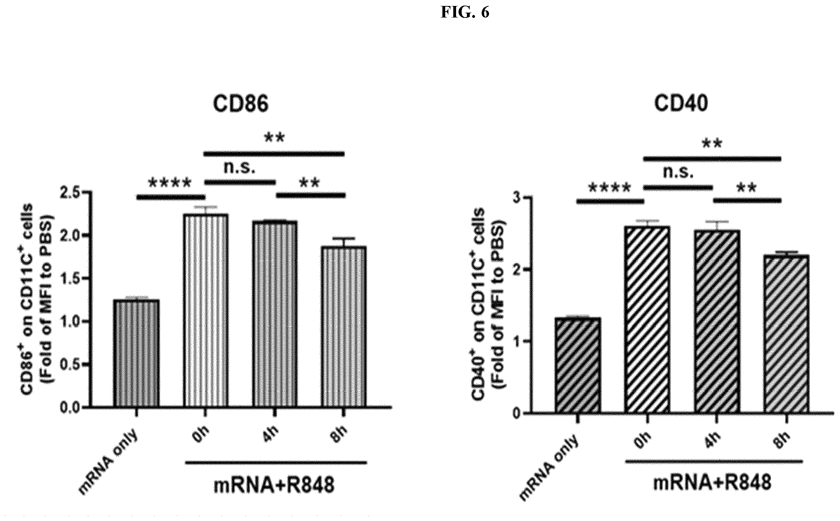

As shown in FIGS. 6, compared with a control treated with mRNA alone (mRNA only), it was confirmed that in an experimental group treated with both mRNA and R848, all cell surface molecules were increased regardless of the difference in treatment time, confirming that the degree of cell activation can increase through the co-administration of mRNA and a toll-like receptor agonist.

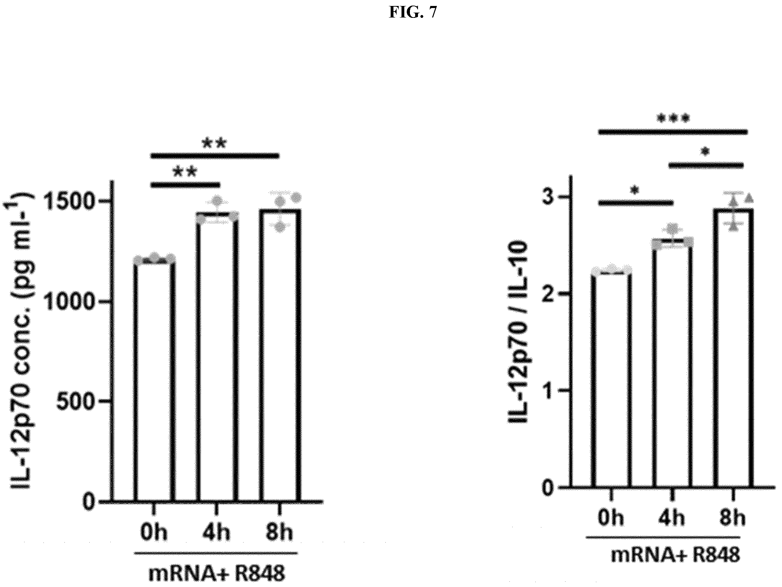

As shown in FIGS. 7, compared with the experimental group (0h) treated with mRNA and R848 at the same time, in the experimental group (4h) treated with both at different times, it was confirmed that the secretion of IL-12p70 increased. In addition, compared to the experimental group (0h) treated with mRNA and R848 at the same time, the ratio (IL-12p70/IL-10) of IL-12p70 and IL-10, used as a Th1 polarization induction indicator, also significantly increased in the experimental groups (4h and 8h) treated with mRNA and R848 at different times.

As shown in FIG. 8, it was confirmed that, in an experimental group (0h) treated with mRNA and LPS at the same time, compared to a control group (mRNA only) treated with mRNA alone, the degree of antigen presentation was reduced, but in experimental groups (4h and 8h) treated with mRNA and then LPS at different times, the degree of antigen presentation was increased.

As shown in FIG. 9, in an experimental group (0h) treated with mRNA and poly I:C at the same time, compared to the control treated with mRNA alone (mRNA only), it was confirmed that, regardless of poly I:C concentration, the degree of antigen presentation was remarkably reduced. On the other hand, in an experimental group (4h) treated with mRNA and poly I:C at different times, regardless of poly I:C concentration (20, 30, and 40), it was confirmed that the degree of antigen presentation was significantly increased.

FIG. 10 shows the result of comparing the tendency of antiviral cytokine, type 1 Interferon (type 1 IFN) after treating mRNA and 40 µg/mL of poly I:C. In the control treated with mRNA alone (mRNA), the secretion of type 1 IFN was not confirmed, but in the experimental group (0h) treated with mRNA and poly I:C at the same time, it was confirmed that the secretion of type 1 IFN markedly increased. In addition, in the experimental group (4h) treated with mRNA and treated with poly I:C four hours later, it was confirmed that the secretion of type 1 IFN was reduced.

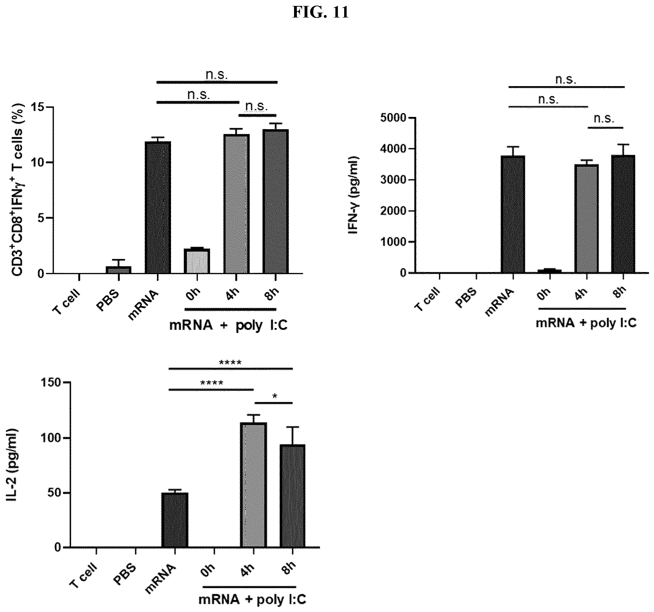

When each dendritic cell (DC) group having different antigen presenting abilities, cell activation degrees, and inflammatory cytokine secretion abilities was co-cultured with T cells isolated from the spleens of ovalbumin-specific TCR transgenic mice (OT-I mice), to confirm the effect of the composition for an mRNA vaccine, the degree of OT-1 T cell activation was confirmed. To confirm the degree of T cell activation, interferon-γ (IFN-γ), which is a cytokine secreted from activated T cells, was confirmed. In further detail, the ratio of cells with IFN-γ in the cells among total T cells and the concentration of IFN-γ secreted to the outside of the cells were measured. In addition, the concentration of interleukin-2 (IL-2), which is a cytokine contributing to increases in differentiation and activity of T cells secreted from T cells, was also confirmed. The result is shown in FIGS. 11.

As shown in FIGS. 11, compared to a control (mRNA) in which BMDCs and T cells treated with mRNA alone, in an experimental group (0h) in which BMDCs and T cells treated with mRNA and poly I:C at the same time, it was confirmed that all of the amount of IFN-γ⁺ T cells, the level of secreted IFN-γ, and the level of IL-2 were remarkably reduced. On the other hand, in an experimental group (4h) in which BMDCs and T cells treated with mRNA and then treated with poly I:C four hours later or an experimental group (8h) in which BMDCs and T cells treated with mRNA and treated with poly I:C eight hours later, all of the amount of IFN-γ⁺ T cells, the level of secreted IFN-γ, and the level of IL-2 markedly increased. Particularly, in the experimental group (4h) treated with mRNA and treated with poly I:C four hours later, it was confirmed that the level of IL-2 secretion was the highest.

### 12.5.2. Effect of co-administration of toll-like receptor 3 agonist and toll-like receptor 7 or 8 agonist

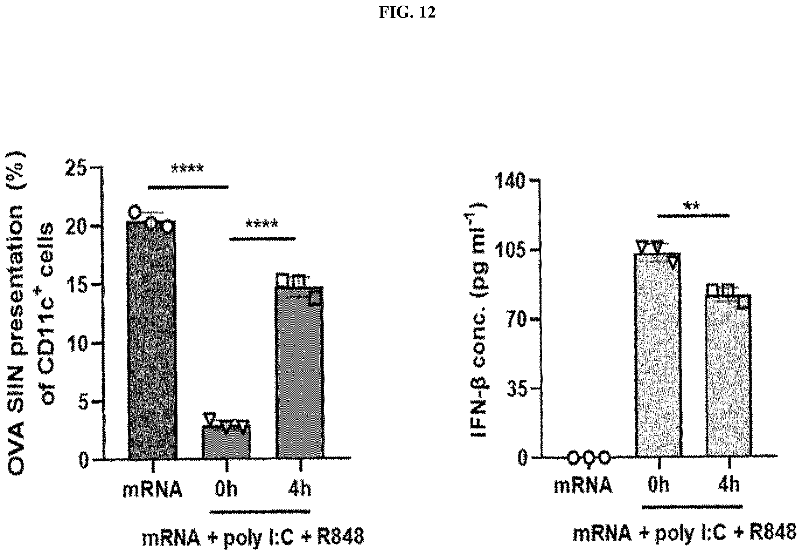

To confirm the effect of the composition for an mRNA vaccine including a combined toll-like receptor, consisting of toll-like receptor 3 agonist and toll-like receptor 7 or 8 agonist, first, BMDCs were treated with poly I:C, R848 and modified-mRNA in the same manner as in Example 12.1, and the degree of antigen presentation and an interferon level were measured. The result is shown in FIGS. 12.

As shown in FIGS. 12, in the case of an experimental group (0h) treated with mRNA and a combined toll-like receptor agonist at the same time, compared with the case treated with mRNA alone, the degree of antigen presentation was remarkably reduced, but on the contrary, in the case of an experimental group (4h) treated with mRNA and treated with a combined toll-like receptor agonist four hours later, it was confirmed that the degree of antigen presentation remarkably increased. In addition, in the control (mRNA) treated with mRNA alone, no secretion of type 1 IFN was identified, and in the case of an experimental group (0h) treated with mRNA and poly I:C and R848 at the same time, it was confirmed that the secretion of type I IFN remarkably increased. In addition, in the case of an experimental group (4h) treated with mRNA and then poly I:C and R848 with a time difference of four hours, it was confirmed that the secretion of type I IFN was reduced.

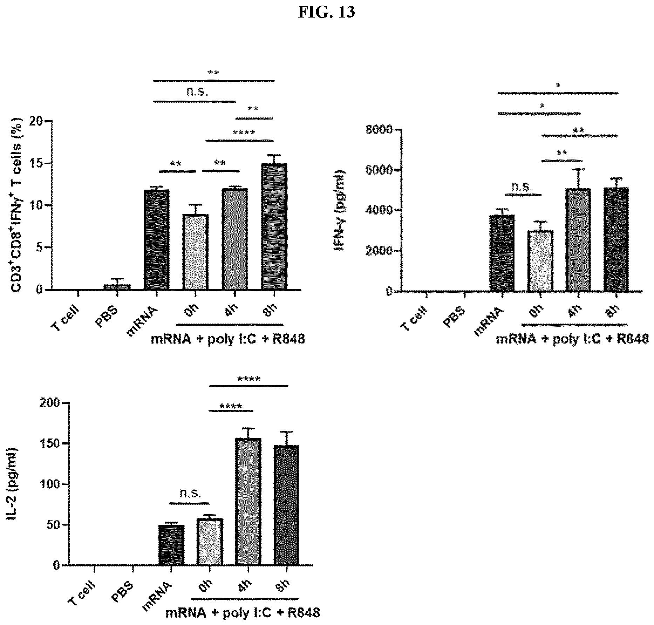

In addition, to confirm the effect of the composition for an mRNA vaccine when BMDCs and T cells were co-cultured, the degree of OT-1 T cell activation was confirmed. The result is shown in FIGS. 13.

As shown in FIGS. 13, compared to a control (mRNA) in which BMDCs and T cells were treated with mRNA alone, an experimental group (0h), in which BMDCs and T cells treated with mRNA, poly I:C, and R848 at the same time, shows similar results, including the amount of IFN-γ⁺ T cells, a level of secreted IFN-γ, and a level of IL-2. On the other hand, in an experimental group (4h) in which BMDCs and T cells were treated with mRNA and treated with poly I:C and R848 four hours later, and, an experimental group (8h) in which BMDCs and T cells were treated with mRNA and treated with poly I:C and R848 eight hours later, it was confirmed that all of the amount of IFN-γ⁺ T cells, the level of secreted IFN-γ, and the level of IL-2 markedly increased.

### 12.5.3. Effect of co-administration of toll-like receptor 4 agonist and toll-like receptor 7 or 8 agonist

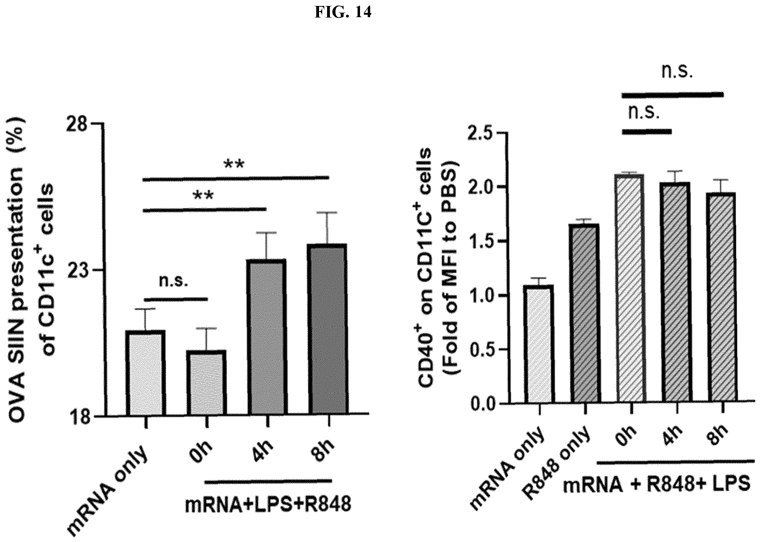

To confirm the effect of the composition for an mRNA vaccine including a combined toll-like receptor, consisting of toll-like receptor 4 agonist and toll-like receptor 7 or 8 agonist, on BMDCs, first, BMDCs were treated with LPS, R848 and modified-mRNA in the same manner as in Example 12.1, and the degree of antigen presentation and the degree of cell activation were confirmed. The degree of cell activation was confirmed by measuring cell surface molecules. The result is shown in FIGS. 14.

As shown in FIGS. 14, compared to the control treated with mRNA alone (mRNA only), in the experimental group (0h) treated with mRNA, LPS and R848 at the same time, the degree of antigen presentation was slightly reduced, but there was no significant difference, in an experimental group (4h) treated with mRNA and treated with LPS and R848 four hours later and in an experimental group (8h) treated with mRNA and treated with LPS and R848 eight hours later, it was confirmed that the degree of antigen presentation remarkably increased, indicating a significant difference. In addition, compared to the control treated with mRNA alone (mRNA only), in the experiment groups treated with mRNA, LPS and R848 together, it was confirmed that cell surface molecules increased.

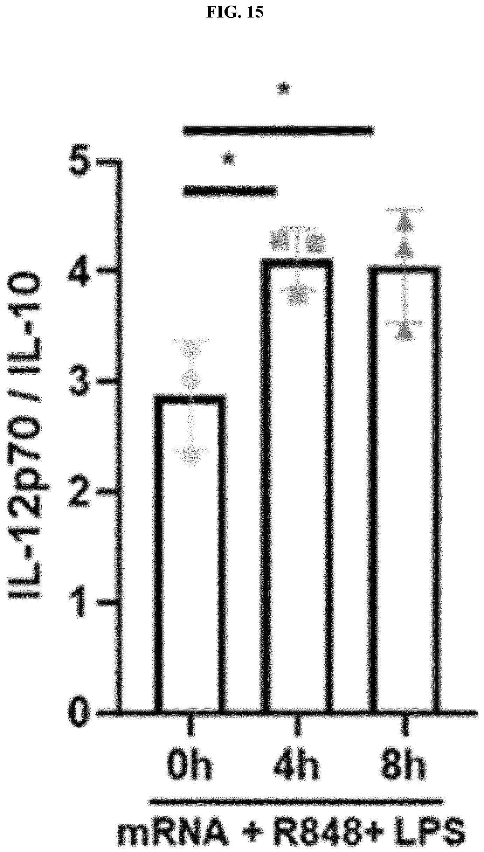

In addition, the ratio of IL-12p70 and IL-10 (IL-12p70/IL-10) used as a Th1 polarization induction indicator was confirmed. The result is shown in FIG. 15.

As shown in FIG. 15, compared with the experimental group (0h) treated with mRNA, LPS and R848 at the same time, in the experimental groups (4h and 8h) treated with the same at different times, it was confirmed that the ratio significantly increased.

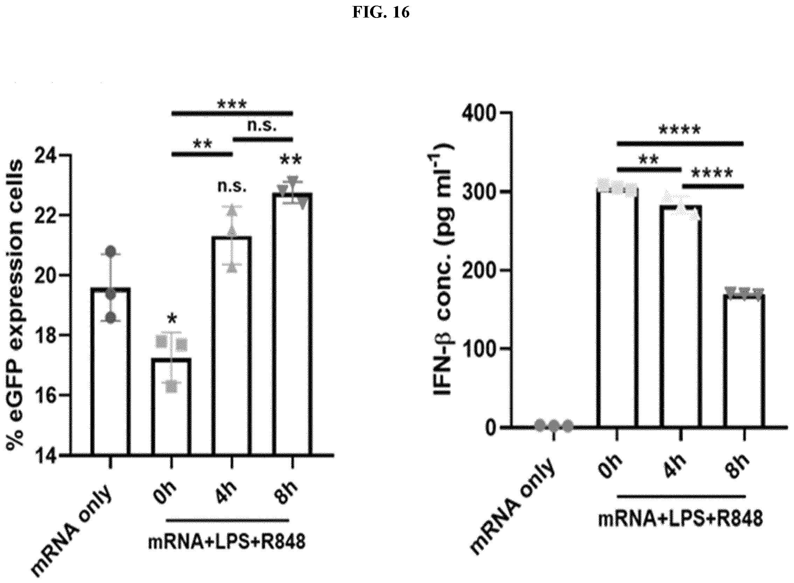

In addition, to confirm the correlation between mRNA and type 1 IFN, 12 hours after BMDCs were treated with eGFP modified-mRNA, the percentage of eGFP protein-expressing cells and a type I IFN level were measured. The result is shown in FIGS. 16.

As shown in FIGS. 16, in the experimental group (0h) treated with mRNA, LPS and R848 at the same time, compared to the control treated with mRNA alone (mRNA only), the number of cells expressing GFP was reduced, but in the experimental groups (4h and 8h) treated at different times, it was confirmed that the number of cells expressing GFP remarkably increased. In addition, in the control treated with mRNA alone, type 1 IFN was not secreted, in the experimental group (0h) treated with mRNA, LPS and R848 at the same time, it was confirmed that the secretion of type 1 IFN was the highest, and as the time interval between treatment of mRNA and toll-like receptor agonists increased, the secretion level was reduced.

From the above results, when an mRNA antigen and a toll-like receptor agonist are administered at the same time, mRNA expression is inhibited, and the immunogenic function of the mRNA antigen is activated, thereby increasing type 1 IFN expression. However, when an mRNA antigen and a toll-like receptor agonist are administered at different times, it was confirmed that, in the beginning, the expression of the mRNA antigen is induced, and then the immunogenicity of the mRNA antigen is also activated at different times.

### 12.6. Confirmation of efficacy of composition for mRNA vaccine on pDCs

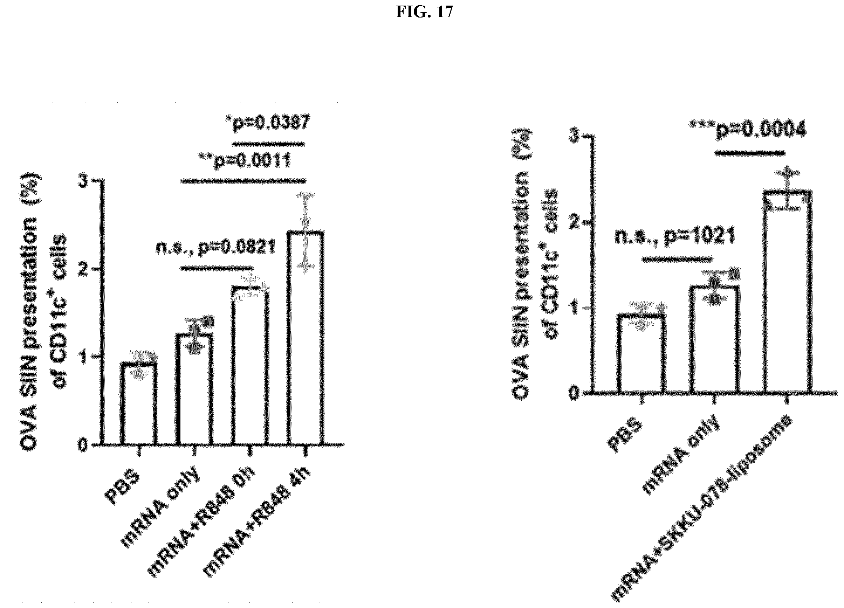

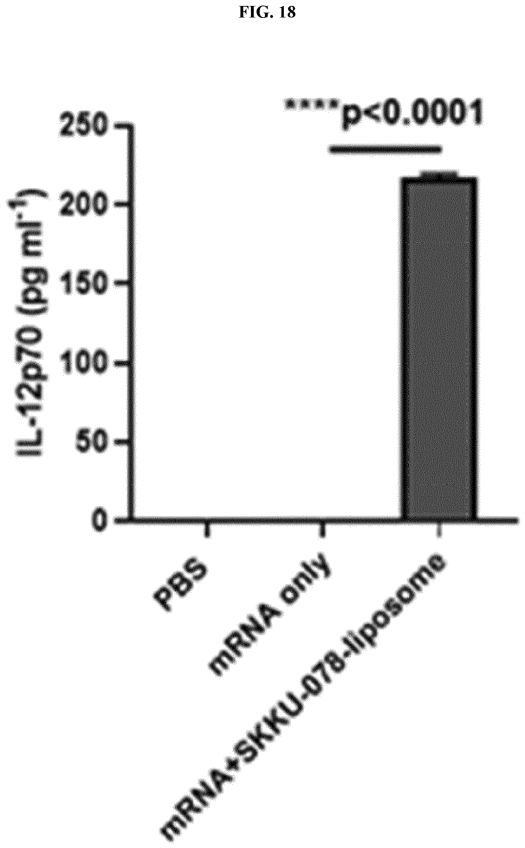

pDCs known to secrete a relatively large amount of type 1 IFN compared to BMDCs were used to perform experiments. The treatment method was performed in the same way as for BMDCs. In addition, as toll-like receptor agonists, a nanoliposome (SKKU-078-liposome) was prepared in the same manner as in Example 11.1 using R848-cholesterol complex in which cholesterol is cross-linked to R848 by a disulfide bond. In addition, the degree of antigen presentation and the amount of interleukin-12p70 secretion were measured. The results are shown in FIGS. 17 and 18.

As shown in FIGS. 17, compared with the control treated with PBS alone (PBS), it was confirmed that the control treated with mRNA alone (mRNA only) does not show significant differences, and in the experimental group (0h) treated with mRNA and R848 at the same time, the degree of antigen presentation slightly increased. However, in the experimental group (4h) treated with mRNA and treated with R848 four hours later, it was confirmed that the degree of antigen presentation significantly increased. In addition, the experimental group treated with a liposome and mRNA at the same time (mRNA+SKKU-078-liposome) also showed a remarkably increased degree of antigen presentation. It was confirmed that this is the same result as that of the experimental group treated with mRNA and treated with R848 after 4 hours.

In addition, as shown in FIG. 18, compared with the control treated with mRNA alone (mRNA only), it was confirmed that, in the experimental group treated with a liposome and mRNA at the same time (mRNA+SKKU-078-liposome), the amount of interleukin-12p70 secretion remarkably increased.

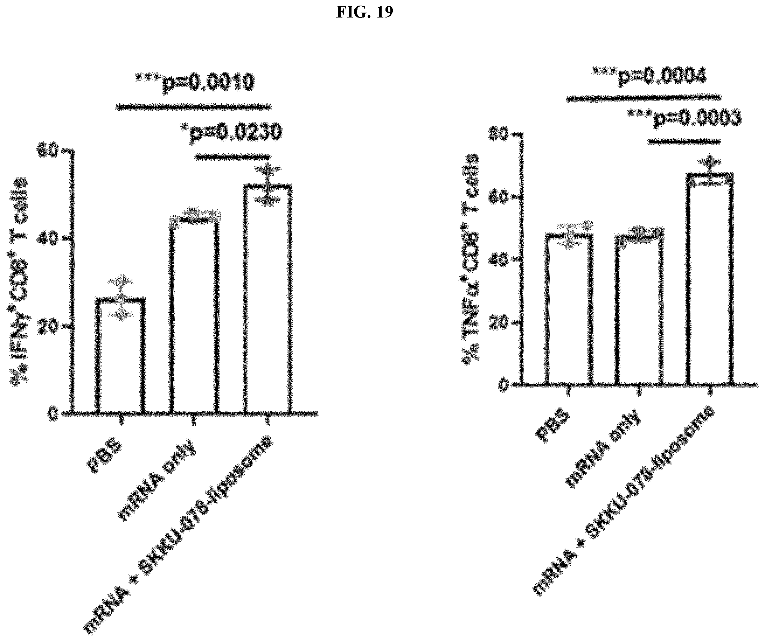

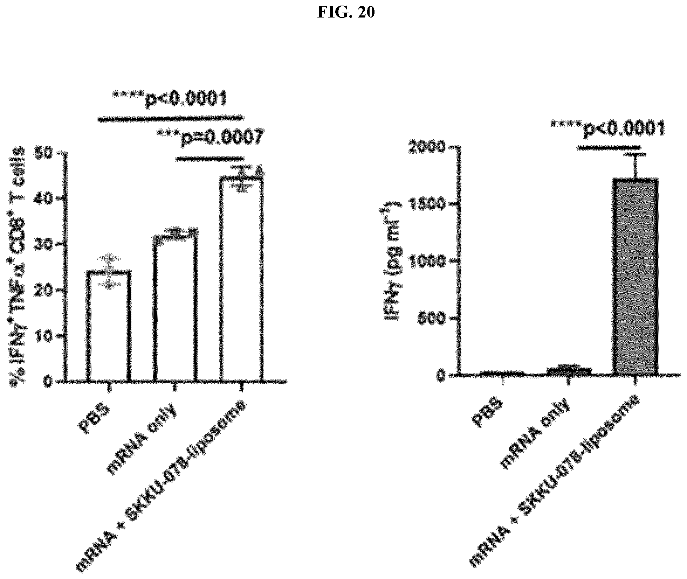

In addition, when pDCs and splenic T cells were co-cultured, to confirm the effect of the composition for an mRNA vaccine, a liposome including modified mRNA and a R848-cholesterol complex was treated. In addition, after 48 hours, the percentage of T cells was analyzed by phenotype. The result is shown in FIGS. 19 and 20.

As shown in FIGS. 19 and 20, it was confirmed that the control treated with mRNA alone (mRNA only) shows a similar or slightly increasing result compared to the PBS-treated control (PBS), but in the experimental group treated with mRNA and a liposome (mRNA+SKKU-078-liposome), it was confirmed that the percentages of interferon gamma (IFN-γ)-positive T cells, tumor necrosis factor alpha (TNF-α)-positive T cells, and dual positive T cells remarkably increased. In addition, it was confirmed that the amount of IFN-γ cytokine secretion also increased.

From the above results, in the preparation of a composition for an mRNA vaccine including a toll-like receptor agonist, when a toll-like receptor agonist and mRNA are injected into the body to work at the same time, the pharmacological activity of mRNA is reduced. On the other hand, after translation of mRNA into a protein, when the activating function of a toll-like receptor agonist, i.e., an adjuvant, sequentially works, it was confirmed that immunization is optimized so the efficacy of the vaccine can be most effectively improved. That is, in the effective preparation of a composition for an mRNA vaccine, it can be confirmed that the sequential working of mRNA and a toll-like receptor agonist is significant. In addition, in the complex of a toll-like receptor agonist and cholesterol of the present invention, since cholesterol is linked to the active site of the toll-like receptor agonist by a cleavable bond, activity is temporarily inhibited at the early stage, but afterward, when the complex reached a desired location due to physiological environments, the toll-like receptor agonist and the cholesterol are separated to exhibit activity, confirming the same effect as that treated with a time difference from mRNA. That is, as the toll-like receptor agonist-cholesterol complex is used as an adjuvant, in the composition for a vaccine including a toll-like receptor agonist-cholesterol complex and mRNA as active ingredients, even with simultaneous administration, translation of RNA into a protein and the activation of the toll-like receptor agonist, which is the agonist, occur in order, confirming that the composition for a vaccine of the present invention can remarkably improve the effect of the composition for an mRNA vaccine, as well as can be widely used in various fields of mRNA vaccines. In addition, as various adjuvants are further added to nanoparticles that can be easily prepared using a toll-like receptor agonist-cholesterol complex, it was confirmed that the immune activation efficacy of the composition for an mRNA vaccine can be further improved.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

Since, in an mRNA vaccine including an adjuvant whose immune activating function is kinetically controlled, the adjuvant is activated after the induction of mRNA translation, even with simultaneous administration, the translation of mRNA into a protein and the activating function of a toll-like receptor agonist, which is the adjuvant, occur sequentially, and thus the effect of a composition for an mRNA vaccine can be remarkably enhanced. Therefore, the composition for an mRNA vaccine in the present invention can be widely used in various fields of mRNA vaccines.

## Claims

1. A composition for an mRNA vaccine, comprising mRNA antigen and a kinetically controllable immune modulator as active ingredients,
wherein the immune modulator is a complex in which a cleavable linker binds to the active site of an immune modulator.

2. The composition of claim 1, wherein, after administration of the composition for an mRNA vaccine, translation of mRNA into a protein proceeds, and sequentially the cleavable linker binding to the active site of the immune modulator is cleaved to induce the activation function of the immune modulator.

3. The composition of claim 1, wherein the kinetic control appears in the manner that when the cleavable linker is linked to the active site of the immune modulator to maintain an inactive state, and then the cleavable linker blocking the active site is cleaved within 2 to 12 hours after mRNA translation has begun, the activity of the immune modulator is temporally delayed.

4. The composition of claim 1, wherein the cleavable linker comprises any one or more bonds selected from the group consisting of disulfide, carbamate, hydrazine, ester, peptide, azide, amide, hydrazone, thioether, phosphodiester, thioketal bonds, and a combination thereof.

5. The composition of claim 1, wherein the cleavable linker further comprises ethylene oxide or ethylene glycol at one or both ends thereof.

6. The composition of claim 1, wherein the cleavable linker is cleaved at the chemical bond of a binding site due to any one or more factors selected from the group consisting of an enzyme, pH, redox potential, a temperature, ultrasonic wave, magnetic force, and a light source.

7. The composition of claim 1, wherein, to an end of the cleavable linker, any one or more materials selected from the group consisting of cholesterol, a lipid, a protein, an amino acid, a peptide and an oligonucleotide is bound.

8. The composition of claim 1, wherein the immune modulator is a toll-like receptor agonist.

9. The composition of claim 8, wherein the toll-like receptor agonist is any one or more selected from the group consisting of a toll-like receptor 1 agonist, a toll-like receptor 2 agonist, a toll-like receptor 3 agonist, a toll-like receptor 4 agonist, a toll-like receptor 5 agonist, a toll-like receptor 6 agonist, a toll-like receptor 7 or 8 agonist, and a toll-like receptor 9 agonist.

10. The composition of claim 1, wherein the mRNA antigen and the kinetically controllable immune modulator are loaded into any one or more drug delivery systems selected from the group consisting of a nanoliposome, a nanoemulsion, a nanomicelle, a hydrogel, a scaffold, a solid nanoparticle, and a polymeric nanoparticle.

11. The composition of claim 10, wherein, in the drug delivery system, after delivering the mRNA antigen loaded in the drug delivery systems to the cytosol first, the kinetically controllable immune modulator interacts with a receptor on a cell surface, or in an endosome or lysosome.

12. The composition of claim 10, the drug delivery system further comprises any one or more immune modulators selected from the group consisting of a toll-like receptor agonist, saponin, an antiviral peptide, an inflammasome inducer, a NOD ligand, a cytosolic DNA sensor (CDS ligand), a stimulator of interferon genes (STING) ligand, an outer wall component of a pathogen, alum, a lipid, a combination thereof, and a derivative thereof.

13. The composition of claim 1, wherein the composition for an mRNA vaccine is used for prevention or treatment of any one or more diseases selected from the group consisting of an infectious disease, cancer, metabolic syndrome, an autoimmune disease, and a rare disease.

14. A method of preventing or treating an infectious disease, cancer, metabolic syndrome, an autoimmune disease, or a rare disease, comprising:
administering a composition for an mRNA vaccine comprising an mRNA antigen and a kinetically controllable immune modulator as active ingredients to a subj ect.

15. A composition for an mRNA vaccine for use in preventing or treating an infectious disease, cancer, metabolic syndrome, an autoimmune disease, or a rare disease, the composition comprising an mRNA antigen and a kinetically controllable immune modulator as active ingredients.

16. A use of a composition for an mRNA vaccine comprising an mRNA antigen and a kinetically controllable immune modulator as active ingredients for the manufacture of medicament for preventing or treating of an infectious disease, cancer, metabolic syndrome, an autoimmune disease, or a rare disease.
